# EUROPÄISCHE PATENTSCHRIFT

(11) **EP 1 558 257 B1**
(45) Veröffentlichungstag und Bekanntmachung des Hinweises auf die Patenterteilung: **25.12.2013**
(21) Anmeldenummer: 03769447.8
(22) Anmeldetag: 24.10.2003
(51) Int. Cl.: A61K 31/485, A61K 31/4725, A61K 9/20

(54) **GEGEN MISSBRAUCH GESICHERTE DARREICHUNGSFORM**
DOSAGE FORM THAT IS SAFEGUARDED FROM ABUSE
FORME GALENIQUE PROTEGEE CONTRE UN USAGE DETOURNE

(30) Priorität: 25.10.2002 DE 10250084
(43) Veröffentlichungstag der Anmeldung: 03.08.2005
(73) Patentinhaber: Grünenthal GmbH, 52078 Aachen (DE)
(72) Erfinder: BARTHOLOMÄUS, Johannes, 52080 Aachen (DE); KUGELMANN, Heinrich, 52068 Aachen (DE); LANGNER, Klaus-Dieter, 52078 Aachen (DE)
(74) Vertreter: Kutzenberger, Helga
(86) Internationale Anmeldenummer: PCT/EP2003/011784
(87) Internationale Veröffentlichungsnummer: WO 2004/037259

(56) Entgegenhaltungen:
- EP-A- 1 201 233
- EP-A- 1 293 195
- WO-A-97/33566
- WO-A-02/094254
- WO-A-03/018015
- WO-A-03/039561
- US-A- 3 980 766
- US-A- 4 070 494
- US-A- 4 175 119
- US-A- 5 149 538
- US-B1- 6 228 863
- US-B1- 6 309 668

## Beschreibung

Die vorliegende Erfindung betrifft eine gegen Mißbrauch gesicherte Darreichungsform, die neben einem oder mehreren Wirkstoffen mit Mißbrauchspotential zwei oder mehrere der nachfolgenden Komponenten a)-d) aufweist:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wäßrigen Flüssigkeit aus der Darreichungsform gewonnenen Extrakt ein Gel bildet, welches beim Einbringen in eine weitere Menge einer wäßrigen Flüssigkeit visuell unterscheidbar bleibt,
(c) wenigstens einen Antagonisten für den Wirkstoff bzw. die Wirkstoffe mit Mißbrauchspotential,
(d) wenigstens ein Emetikum.

Eine Vielzahl von pharmazeutischen Wirkstoffen weist neben einer ausgezeichneten Wirksamkeit auf ihrem betreffenden Anwendungsgebiet auch ein Mißbrauchspotential auf, d.h. sie können von einem Mißbraucher eingesetzt werden, um Wirkungen herbeizuführen, die nicht ihrem medizinischen Bestimmungszweck entsprechen.
So werden beispielsweise Opiate, die eine exzellente Wirksamkeit bei der Bekämpfung von starken bis sehr starken Schmerzen zeigen, von Mißbrauchern häufig zum Erzielen rauschartiger, euphorisierender Zustände verwendet.

Orale Darreichungsformen, die solche Wirkstoffe mit Mißbrauchspotential enthalten, führen üblicherweise selbst bei der Einnahme mißbräuchlich hoher Mengen nicht zu dem vom Mißbraucher gewünschten Ergebnis, da die Wirkstoffe im Blut nur langsam anfluten. Um dennoch einen Mißbrauch zu ermöglichen, werden die entsprechenden Darreichungsformen vom Mißbraucher zerkleinert, z.B. gemörsert, und z.B. durch Schnupfen über die Nase appliziert. Bei einer weiteren Form des Mißbrauchs wird der Wirkstoff aus dem durch Zerkleinerung der Darreichungsform erhaltenen Pulver mit Hilfe einer vorzugsweise wäßrigen Flüssigkeit extrahiert und die resultierende Lösung, ggf. nach Filtration durch Watte oder Zellstoff, parenteral, insbesondere intravenös, appliziert. Bei diesen Formen der Applikation kommt es zu einem gegenüber der oralen Applikation beschleunigten Anfluten des Wirkstoffes mit dem vom Mißbraucher gewünschten Ergebnis.

Die Aufgabe der vorliegenden Erfindung bestand daher darin, eine Darreichungsform für Wirkstoffe mit Mißbrauchspotential zur Verfügung zu stellen, die bei bestimmungsgemäßer Applikation deren therapeutische Wirkung gewährleistet, bei einer mißbräuchlichen Einnahme jedoch nicht die vom Mißbraucher gewünschte Wirkung entfalten.

Diese Aufgabe wurde durch die erfindungsgemäße, gegen Mißbrauch gesicherte Darreichungsform gelöst, die neben einem oder mehreren Wirkstoffen mit Mißbrauchspotential zwei oder mehr der nachfolgenden Komponenten a)-d) aufweist:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wäßrigen Flüssigkeit aus der Darreichungsform gewonnenen Extrakt ein Gel bildet, welches beim Einbringen in eine weitere Menge einer wäßrigen Flüssigkeit visuell unterscheidbar bleibt,
(c) wenigstens einen Antagonisten für den Wirkstoff bzw. die Wirkstoffe mit Mißbrauchspotential,
(d) wenigstens ein Emetikum.

Die Komponenten (a) bis (d) sind jeweils für sich allein bereits zur Sicherung der Darreichungsform gegen Mißbrauch geeignet. (siehe z.B. EP1201233, US 5149538, WO 97/33566, US 6228863, US 3980766, US 4070494, US 6309668, US 4175119, WO 02/094254, WO 03/018015, EP 1293195, WO 03/039561). So eignet sich die Komponente (a) bevorzugt zur Sicherung gegen nasalen und/oder parenteralen Mißbrauch, die Komponente (b) bevorzugt gegen parenteralen Mißbrauch, die Komponente (c) bevorzugt gegen nasalen und/oder parenteralen Mißbrauch und die Komponente (d) vorzugsweise gegen parenteralen und/oder oralen und/oder nasalen Mißbrauch. Durch die erfindungsgemäße Kombination von wenigstens zwei dieser vorstehend genannten Komponenten, gelingt es, die erfindungsgemäße Darreichungsform noch effektiver gegen Mißbrauch zu schützen.

In einer Ausführungsform weist die erfindungsgemäße Darreichungsform drei der Komponenten (a)-(d) in der Missbrauchskombination auf, vorzugsweise (a), (b) und (c) oder (a), (b) und (d).

In einer weiteren Ausführungsform weist die erfindungsgemäße Darreichungsform sämtliche Komponenten (a)-(d) auf.

Pharmazeutische Wirkstoffe mit Mißbrauchspotential sind dem Fachmann, ebenso wie die einzusetzenden Mengen und Verfahren zu ihrer Herstellung. an sich bekannt und können als solche in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Eine Kombination von zwei oder drei der Komponenten (a), (b) und (d) eignet sich besonders zur Verhinderung des Mißbrauchs eines pharmazeutischen Wirkstoffs, der ausgewählt ist aus der Gruppe bestehend aus Opiaten, Opioiden, Tranquillantien, vorzugsweise Benzodiazepinen, Stimulantien und weiteren Betäubungsmitteln.

Besonders eignet sich eine Kombination von zwei oder drei der Komponenten (a), (b) und (d) zur Verhinderung des Mißbrauchs von Opiaten, Opioiden, Tranquillantien sowie weitere Betäubungsmitteln, die ausgewählt sind aus der Gruppe bestehend aus N-{ 1-[2-(4-Ethyl-5-oxo-2-tetrazolin-1-yl)ethyl]-4-methoxymethyl-4-piperidyl}propionanilid (Alfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-α-Methylphenethylamin (Amfetamin), 2-(α-Methylphenethylamino)-2-phenylacetonitril

(Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3*H*)-on (Bromazepam), 2-Brom-4-(2-chlorphenyl)-9-methyl-6*H*-thieno[3,2-*f*][1,2,4]triazolo[4,3-a][1,4]diazepin (Brotizolam), 17-Cyclopropylmethyl-4,5α-epopxy-7α[(S)-1-hydroxy-1,2,2-trimethyl-propyl]-6-methoxy-6,14-*endo-*ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1*S*,2*S*)-2-Amino-1-phenyl-1-propanol (Cathin / D-Norpseudoephedrin), 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*)-dion (Clobazam), 5-(2-Chlorphenyl)-7-nitro-1*H*-1,4-benzodiazepin-2(3*H*)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H-*thieno[2,3-e][1,4]diazepin-2(3*H*)-on (Clotiazepam), 10-Chlor-11b-(2chlorphenyl)-2,3,7,11b-tetrahydrooxazolo[3,2-*d*][1,4]benzodiazepin-6(5*H*)-on (Cloxazolam), (-)-Methyl-[3β-benzoyloxy-2β(1α*H*,5α*H*)-tropancarboxylat] (Cocain), 4,5α-Epoxy-3-methoxy-17-methyl-7-morphinen-6α-ol (Codein), 5-(1-Cyclohexenyl)-5-ethylbarbitursäure (Cyclobarbital), Cyclorphan, Cyprenorphin, 7-Chlor-5-(2-chlorphenyl)-1*H*-1,4-benzodiazepin-2(3*H*)-on (Delorazepam), Desomorphin, Dextromoramid, (+)-(1-Benzyl-3-dimethylamino-2-methyl-1-phenylpropyl)propionat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6a*R*,10a*R*)-6,6,9-Trimethyl-3-pentyl-6a,7,8,10a-tetrahydro-6*H*-benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethylloflazepat), 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-*endo*-etheno-morphinan-3-ol (Etorphin), *N-*Ethyl-3-phenyl-8,9,10-trinorbornan-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1*H*-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1*H*-1,4-benzodiazepin-2(3*H*)-on (Halazepam), 10-Brom-11 b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3*S*,6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 6-(2-Chlorphenyl)-2-(4-methyl-1-piperazinylmethylen)-8-nitro-2*H-*imidazo[1,2-a][1,4] benzodiazepin-1(4*H*)-on (Loprazolam), 7-Chlor-5-(2-chlorphenyl)-3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H*-imidazo[2,1-a]isoindol-5-ol (Mazindol), 7-Chlor-2,3-dihydro-1-methyl-5-phenyl-1*H*-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-*o*-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-*a*][1,4]benzodiazepin (Midazolam), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,10α-tetrahydro-1-hydroxy-6,6-dimethyl-6*H*-dibenzo [*b*, *d*]pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*)-on (Oxazepam), (*cis*-*trans*)-10-Chlor-2,3,7,11b-tetrahydro-2-methyl-11b-phenyloxazolo[3,2-*d*][1,4] benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pernolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-phenyl-1*H*-1,4-benzodiazepin-2(3*H*)-on (Prazepam), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), 5-sec-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3H)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1R*,2R*)-3-(3-Dimethylamino-1-ethyl-2-methyl-propyl)-phenol, (1R, 2R, 4S)-2-[Dimethylamino)methyl-4-(p-fluorbenzyloxy)-1-(m-methoxyphenyl)cyclohexanol, jeweils ggf. in Form entsprechender stereoisomerer Verbindungen sowie entsprechender Derivate, insbesondere Ester oder Ether, und jeweils physiologisch verträglicher Verbindungen, insbesondere Salze und Solvate. Sofern die Kombination zur Sicherung gegen Mißbrauch die Sicherung durch die Komponente (c) umfaßt, eignet sie sich besonders zur Verhinderung des Mißbrauchs eines pharmazeutischen Wirkstoffs, der ausgewählt ist aus der Gruppe bestehend aus Opiaten, Opioiden, Stimulantien und weiteren Betäubungsmitteln.

Besonders geeignet ist eine die Komponente (c) umfassende Kombination zur Verhinderung des Mißbrauchs von Opiaten, Opioiden sowie weiteren Betäubungsmitteln, die ausgewählt ist aus der Gruppe bestehend aus N-{1-[2-(4-Ethyl-5-oxo-2-tetrazolin-1-yl)ethyl]-4-methoxymethyl-4-piperidyl}propionanilid (Alfentanil), Allylprodin, Alphaprodin, 2-Diethylaminopropiophenon (Amfepramon), (±)-α-Methylphenethylamin (Amfetamin), 2-(α-Methylphenethylamino)-2-phenylacetonitril (Amfetaminil), Anileridin, Apocodein, Benzylmorphin, Bezitramid, 17-Cyclopropylmethyl-4,5α-epopxy-7α[(S)-1-hydroxy-1,2,2-trimethyl-propyl]-6-methoxy-6,14-endo-ethanomorphinan-3-ol (Buprenorphin), Butorphanol, (1*S*,2*S*)-2-Amino-1-phenyl-1-propanol (Cathin / D-Norpseudoephedrin), Clonitazen, (-)-Methyl-[3β-benzoyloxy-2β(1α*H*,5α*H*)-tropancarboxylat] (Cocain), 4,5α-Epoxy-3-methoxy-17-methyl-7-morphinen-6α-ol (Codein), Cyclorphan, Cyprenorphin, Desomorphin, Dextromoramid, (+)-(1-Benzyl-3-dimethylamino-2-methyl-1-phenylpropyl)propionat (Dextropropoxyphen), Dezocin, Diampromid, Diamorphon, 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6a*R*,10a*R*)-6,6,9-Trimethyl-3-pentyl-6a,7,8,10a-tetrahydro-6*H*-benzo[c]chromen-1-ol (Dronabinol), Eptazocin, Ethoheptazin, Ethylmethylthiambuten, 4,5α-Epoxy-3-ethoxy-17-methyl-7-morphinen-6□-ol (Ethylmorphin), Etonitazen, 4,5α-Epoxy-7α-(1 -hydroxy-1-methylbutyl)-6-methoxy-17-methyl-6,14-*endo*-etheno-morphinan-3-ol (Etorphin), *N*-Ethyl-3-phenyl-8,9,10-trinorbornan-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 1-[4-(3-Hydroxyphenyl)-1-methyl-4-piperidyl]-1-propanon (Ketobemidon), (3S,6S)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 5-(4-Chlorphenyl)-2,5-dihydro-3*H*-imidazo[2,1-a]isoindol-5-ol (Mazindol), *N*-(3-Chlorpropyl)-α-methylphenethylamin (Mefenorex), Meperidin, Meptazinol, Metazocin, Methylmorphin, N,α-Dimethylphenethylamin (Metamfetamin), (±)-6-Dimethylamino-4,4-diphenyl-3-heptanon (Methadon), Methyl-[2-phenyl-2-(2-piperidyl)acetat] (Methylphenidat), 3,3-Diethyl-5-methyl-2,4-piperidindion (Methyprylon), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,10α-tetrahydro-1-hydroxy-6,6-dimethyl-6*H*-dibenzo [*b*, *d*]pyran-9(6α*H*)-on (Nabilon), Nalbuphen, Narcein, Nicomorphin, Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pernolin), 1,2,3,4,5,6-Hexahydro-6,11-dimethyl-3-(3-methyl-2-butenyl)-2,6-methano-3-benzazocin-8-ol (Pentazocin), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), α,α-Dimethylphenethylamin (Phentermin), α-(2-Piperidyl)benzhydrylalkohol (Pipradrol), 1'-(3-Cyan-3,3-diphenylpropyl)[1,4'-bipiperidin]-4'-carboxamid (Piritramid), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), *N*-{4-Methoxymethyl-1-[2-(2-thienyl)ethyl]-4-piperidyl}propionanilid (Sufentanil), Ethyl-(2-dimethylamino-1-phenyl-3-cyclohexen-1-carboxylat) (Tilidin (cis und trans)), Tramadol, (1R*,2R*)-3-(3-Dimethylamino-1-ethyl-2-methyl-propyl)-phenol, (1 R, 2R, 4S)-2-[Dimethylamino)methyl-4-(p-fluorbenzyloxy)-1-(m-methoxyphenyl)cyclohexanol, jeweils ggf. in Form entsprechender stereoisomerer Verbindungen sowie entsprechender Derivate, insbesondere Ester oder Ether, und jeweils physiologisch verträglicher Verbindungen, insbesondere Salze und Solvate.

Die Verbindungen (1R*,2R*)-3-(3-Dimethylamino-1-ethyl-2-methyl-propyl)-phenol und (1 R, 2R, 4S)-2-[Dimethylamino)methyl-4-(p-fluorbenzyloxy)-1-(m-methoxyphenyl)cyclohexanol, deren physiologisch verträglichen Verbindungen, insbesondere deren Hydrochloride, sowie Verfahren zu ihrer Herstellung sind z.B. aus EP-A-693475 bzw. EP-A-780369 bekannt. Die entsprechenden Beschreibungen werden hiermit als Referenz eingeführt und gelten als Teil der Offenbarung.

Die erfindungsgemäße Darreichungsform enthaltend eine Kombination aus wenigstens zwei der Komponenten a)-d) eignet sich auch zur Verhinderung des Mißbrauchs von Stimulanzien, bevorzugt solcher, die ausgewählt sind aus der Gruppe bestehend aus Amphetamin, Norpseudoephedrin, Methylphenidat und jeweils ggf. deren entsprechenden physiologisch verträglichen Verbindungen, insbesondere deren Basen, Salzen und Solvaten.

Sofern die Kombination zur Sicherung der erfindungsgemäßen Darreichungsform gegen Mißbrauch die Komponente (a) umfaßt, kommen als den Nasen- und/oder Rachenraum reizende Stoffe erfindungsgemäß sämtliche Stoffe in Betracht, die brei entsprechender Applikation über den Nasen- und/oder Rachenraum eine Reaktion des Körpers hervorrufen, die entweder für den Mißbraucher so unangenehm ist, daß der die Applikation nicht weiter fortsetzen will oder kann, z.B. ein Brennen, oder die auf physiologische Art und Weise einer Aufnahme des entsprechenden Wirkstoffes entgegenwirken, z.B. über eine vermehrte nasale Sekretbildung oder Niesen. Es wurde zudem gefunden, daß üblicherweise diese den Nasen- und/oder Rachenraum reizenden Stoffe auch bei parenteraler, insbesondere intravenöser, Applikation ein sehr unangenehmes Gefühl bis hin zu unerträglichen Schmerzen verursachen, so daß der Mißbraucher die Einnahme nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff., beschrieben. Die entsprechende Beschreibung wird hiermit als Referenz eingeführt und gilt als Teil der Offenbarung.
Vorzugsweise kann die erfindungsgemäße Darreichungsform die Pflanzenteile der entsprechenden Scharfstoffdrogen in einer Menge von 0,01 bis 30 Gew.-%, besonders bevorzugt 0,1 bis 0,5 Gew.-%, jeweils bezogen auf das Gesamtgewicht der Darreichungseinheit, enthalten.
Kommen ein oder mehrere Inhaltsstoffe entsprechender Scharfstoffdrogen zum Einsatz, beträgt deren Menge in einer erfindungsgemäßen Darreichungseinheit bevorzugt 0,001 bis 0,005 Gew.-%, bezogen auf das Gesamtgewicht der Darreichungseinheit.

Unter Darreichungseinheit wird eine separate bzw. separierbare Dosiseinheit, wie z. B. eine Tablette oder eine Kapsel, verstanden.

Vorzugsweise weist die erfindungsgemäße Darreichungsform als Komponente (a) einen oder mehrere Inhaltsstoffe wenigstens einer Scharfstoffdroge, ausgewählt aus der Gruppe bestehend aus Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe bestehend aus Capsici Fructus (Paprika), Capsici Fructus acer (Cayennepfeffer) und Piperis nigri Fructus (Pfeffer), auf.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist der Inhaltsstoff der Scharfstoffdrogen ausgewählt aus der Gruppe bestehend aus Myristicin, Elemicin, Isoeugenol, β-Asaron, Safrol, Gingerolen, Xanthorrhizol, Capsaicinoiden, vorzugsweise Capsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und von diesen Inhaltsstoffen abgeleiteten Verbindungen.

Eine weitere Möglichkeit, die erfindungsgemäße Darreichungsform zusätzlich gegen Mißbrauch zu sichern, besteht darin, ihr wenigstens ein viskositätserhöhendes Mittel als weitere Mißbrauchsverhindernde Komponente (b) zuzusetzen, das in einem mit Hilfe einer notwendigen Mindestmenge an einer wäßrigen Flüssigkeit aus der Darreichungsform gewonnenen Extrakt ein Gel bildet, welches beim Einbringen in eine weitere Menge einer wäßrigen Flüssigkeit visuell unterscheidbar bleibt.

Visuelle Unterscheidbarkeit im Sinne der vorliegenden Erfindung bedeutet, daß das mit Hilfe einer notwendigen Mindestmenge an wäßriger Flüssigkeit gebildete, Wirkstoff-haltige Gel beim Einbringen vorzugsweise mit einer Injektionsnadel, in eine weitere Menge wäßriger Flüssigkeit von 37°C im wesentlichen unlöslich und zusammenhängend bleibt und nicht auf einfache Weise so dispergiert werden kann, daß eine parenterale, insbesondere intravenöse, gefahrlose Applikation möglich ist. Vorzugsweise beträgt die Dauer der visuellen Unterscheidbarkeit wenigstens eine Minute.

Die Viskositätserhöhung des Extrakts führt dazu, daß dessen Nadelgängigkeit bzw. Spritzbarkeit erschwert oder sogar unmöglich gemacht wird. Des weiteren führt sie dazu, daß der erhaltene Extrakt beim Einbringen in eine weitere Menge wäßriger Flüssigkeit, z.B. durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, daß eine parenterale, insbesondere intravenöse, Applikation gefahrlos möglich ist. In Kombination mit der Komponente (a) und/oder (d) führt dies zusätzlich zu unangenehmen Brennen und/oder Erbrechen.

Eine intravenöse Applikation eines entsprechenden Extraktes würde daher mit großer Wahrscheinlichkeit zur Verstopfung von Gefäßen, verbunden mit schweren Embolien bis hin zum Tod des Mißbrauchers führen.

Zur Überprüfung, ob ein viskositätserhöhendes Mittel als Komponente (b) zur Anwendung in der erfindungsgemäßen Darreichungsform geeignet ist, wird dieses in einer entsprechenden Darreichungsform formuliert, die so erhaltene Darreichungsform zerkleinert, vorzugsweise gemörsert, und mit 10 ml Wasser bei einer Temperatur von 25 °C extrahiert. Bildet sich hierbei ein Gel, welches den obenstehend genannten Bedingungen genügt, eignet sich das entsprechende viskositätserhöhende Mittel zur Herstellung einer erfindungsgemäßen Darreichungsform.

Sofern in der erfindungsgemäßen Darreichungsform eine Mißbrauchssicherung durch eine Kombination enthaltend die Komponente (b) vorgesehen ist, kommen vorzugsweise eine oder mehrere viskositätserhöhende Mittel zum Einsatz, die ausgewählt sind aus der Gruppe bestehend aus mikrokristalliner Cellulose mit 11 Gew.-% Carboxymethylcellulose-Natrium (Avicel^{®} RC 591), Carboxymethylcellulose-Natrium (Blanose^{®}, CMC-Na C300P^{®}, Frimulsion BLC-5^{®}, Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Citrus-Pectin (Cesapectin^{®} HM Medium Rapid Set), Wachsmaisstärke (C*Gel 04201^{®}), Natriumalginat (Frimulsion ALG (E401)^{®}), Guarkernmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150 ^{®}), Tarakernmehl (Polygum 43/1^{®}), Propylenglykoalginat (Protanal-Ester SD-LB^{®}), Apfelpektin, Pektin aus Zitronenschale, Natrium-Hyaluronat, Tragant, Taragummi (Vidogum SP 200^{®}), fermentiertes Polysaccharid- Welan Gum (K1A96), Xanthan-Gummi (Xantural 180^{®}). Die in Klammern angegebenen Bezeichnungen sind die Handelsnamen, unter denen die jeweiligen Materialien am Markt geführt sind. Im allgemeinen ist eine Menge von 0,1 bis 5 Gew.% der/des genannten viskositätserhöhenden Mittels ausreichend, um die vorstehend genannten Bedingungen zu erfüllen.

Die viskositätserhöhenden Mittel der Komponente (b), sofern vorgesehen, liegen in der erfindungsgemäßen Darreichungsform bevorzugt in Mengen von ≥ 5 mg pro Darreichungseinheit, d.h. pro Dosiereinheit vor.

In einer besonders bevorzugten Ausführungsform der vorliegenden Erfindung kommen in der missbrauchssichernden Kombination als Komponente (b) solche viskositätserhöhenden Mittel zum Einsatz, die bei der Extraktion aus der Darreichungsform mit der notwendigen Mindestmenge an wäßriger Flüssigkeit ein Gel bilden, das Luftblasen einschließt. Die so erhaltenen Gele zeichnen sich durch ein trübes Erscheinungsbild aus, durch das der potentielle Mißbraucher zusätzlich optisch gewarnt und von dessen parenteraler Applikation abgehalten wird.

Überraschenderweise ist es möglich, die Wirkstoffe und zumindest das viskositätserhöhende Mittel ohne räumliche Trennung voneinander in der erfindungsgemäßen Darreichungsform zu kombinieren, ohne daß die Freisetzung des Wirkstoffs bei bestimmungsgemäßer Applikation der Darreichungsform gegenüber einer entsprechenden Darreichungsform, die das viskositätserhöhende Mittel nicht aufweist, beeinträchtigt wird.
Selbstverständlich ist es aber auch möglich, die viskositätserhöhenden Mittel und die Wirkstoffe in räumlich voneinander getrennter Anordnung in der Darreichungsform zu kombinieren, wie nachstehend beschrieben.

Des weiteren kann die erfindungsgemäße Darreichungsform in der Kombination zur Sicherung gegen Mißbrauch die Komponente (c) aufweisen, nämlich einen oder mehrere Antagonisten für den Wirkstoff bzw. die Wirkstoffe mit Missbrauchspotential, wobei die Antagonistenmenge räumlich getrennt von Wirkstoff und der Komponente (a) und/oder (b) vorzugsweise vorliegen und keine Wirkung bei bestimmungsgemäßer Verwendung entfalten sollte.

Geeignete Antagonisten zur Verhinderung des Mißbrauchs der Wirkstoffe sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Sofern der in der Darreichungsform vorliegende Wirkstoff ein Opiat oder ein Opioid ist, kommt als Antagonist bevorzugt ein Antagonist ausgewählt aus der Gruppe bestehend aus Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten, sofern eine Ausrüstung mit der Komponente (c) vorgesehen ist, in einer Menge von ≥ 10 mg, besonders bevorzugt in einer Menge von 10 bis 100 mg, ganz besonders bevorzugt in einer Menge von 10 bis 50 mg auf pro Darreichungsform, d.h. pro Dosiereinheit eingesetzt.

Weist die erfindungsgemäße Darreichungsform als Wirkstoffe ein Stimulanz auf, ist der Antagonist bevorzugt ein Neuroleptikum, vorzugsweise ausgewählt aus der Gruppe bestehend aus Haloperidol, Promethacin, Fluphenazin Perphenazin, Levomepromazin, Thioridazin, Perazin, Chlorpromazin, Chlorprothixin, Zuclopentixol, Flupentexol, Prothipendyl, Zotepine, Benperidol, Pipamperon, Melperon und Bromperidol.
Vorzugsweise weist die erfindungsgemäße Darreichungsform diese Antagonisten in einer üblichen, dem Fachmann bekannten therapeutischen Dosierung, besonders bevorzugt in einer gegenüber der üblichen Dosierung verdoppelten bis verdreifachten Menge pro Dosiereinheit auf.

Sofern die Kombination zur Sicherung der erfindungsgemäßen Darreichungsform gegen Mißbrauch die Komponente (d) umfaßt, kann sie wenigstens ein Emetikum aufweisen, das vorzugsweise in einer räumlich getrennten Anwendung von der gegebenbenenfalls vorhandenen Komponente (a) und/oder (b) und dem Wirkstoff vorliegen und bei bestimmungsgemäßer Anwendung kleine Wirkung im Körper entfalten sollte.

Geeignete Emetika zur Verhinderung des Mißbrauchs der Wirkstoffe sind dem Fachmann an sich bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäßen Darreichungsform vorliegen.

Sofern die Kombination zur Sicherung gegen Mißbrauch die Komponente (d) enthält, kommt in der erfindungsgemäßen Darreichungsform bevorzugt ein Emetikum auf Basis eines oder mehrerer Inhaltsstoffe von Radix Ipecacuanhae (Brechwurzel), vorzugsweise auf Basis des Inhaltsstoffes Emetin, in Betracht, wie sie z.B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden. Die entsprechende Literaturbeschreibung wird hiermit als Referenz eingeführt und gilt als Teil der Offenbarung.

Vorzugsweise kann die erfindungsgemäße Darreichungsform als Komponente (d) das Emetikum Emetin aufweisen, bevorzugt in einer Menge von ≥ 10 mg, besonders bevorzugt ≥ 20 mg und ganz besonders bevorzugt in einer Menge von ≥ 40 mg pro Darreichungsform, d.h. Dosiereinheit.

Ebenfalls bevorzugt kann als Emetikum Apomorphin in der erfindungsgemäßen Missbrauchssicherung zum Einsatz kommen, vorzugsweise in einer Menge von vorzugsweise ≥ 3 mg, besonders bevorzugt ≥ 5 mg und ganz besonders bevorzugt ≥ 7 mg pro Dosiereinheit.

Die Formulierung der erfindungsgemäßen Darreichungsform kann in vielfältiger Art und Weise nach üblichen, dem Fachmann bekannten Methoden erfolgen. Methoden zur Formulierung der Darreichungsform sind dem Fachmann bekannt, beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang Gerhart, 1. Auflage, 1998, Medpharm Scientific Publishers. Die entsprechende Beschreibung wird hiermit als Referenz eingeführt und gilt als Teil der Offenbarung.

Vorzugsweise eignen sich die erfindungsgemäßen Darreichungsformen zur oralen Applikation.

In einer bevorzugten Ausführungsform liegt die erfindungsgemäße Darreichungsform in Form einer Tablette, einer Kapsel oder in Form eines oralen osmotischen therapeutischen Systems (OROS) vor.

Eine besonders einfache Art der Formulierung der erfindungsgemäßen Darreichungsform besteht darin, zwei oder mehr der Komponenten (a)-(d) mit dem Wirkstoff bzw. den Wirkstoffen und ggf. physiologisch verträglichen Hilfsstoffen zu mischen und dieses Gemisch in eine Kapsel abzufüllen oder zu einer Tablette zu verpressen, sofernim Hinblick auf die Komponenten (c) bzw. (d) bei der bestimmungsgemäßen, oralen Applikation die Toleranzgrenzen eingehalten werden. Bei dieser Art der Formulierung der Darreichungsform ist darauf zu achten, daß die Komponenten (c) und/oder (d) so formuliert oder so gering dosiert sind, daß sie bei bestimmungsgemäßer Applikation der Darreichungsform praktisch keine den Patienten oder die Wirksamkeit des Wirkstoffs beeinträchtigende Wirkung entfalten können.

In einer weiteren bevorzugten Ausführungsform enthält die erfindungsgemäße Darreichungsform die Komponente (d) in einer Dosierung, die so gewählt ist, daß bei bestimmungsgemäßer oraler Applikation keine negative Wirkung hervorgerufen wird. Wird jedoch die vorgesehene Dosierung der Darreichungsform versehentlich, insbesondere durch Kinder, oder beim Mißbrauch überschritten, wird Übelkeit bzw. Brechreiz hervorgerufen. Die jeweilige Menge der Komponente (d), die vom Patienten bei bestimmungsgemäßer oraler Applikation noch toleriert wird, kann vom Fachmann durch einfache Vorversuche ermittelt werden.

Orale osmotische therapeutische Systeme sowie geeignete Materialien und Verfahren zu ihrer Herstellung sind dem Fachmann an sich bekannt, beispielsweise aus US 4,612,008, US 4,765,989 und US 4,783,337. Die entsprechenden Beschreibungen werden hiermit als Referenz eingeführt und gelten als Teil der Offenbarung.

Sofern aber eine Kombination zur Sicherung der Darreichungsform enthaltend die Komponenten (c) und/oder (d) vorgesehen ist, sollten diese Komponenten bevorzugt in einer so hohen Dosierung zum Einsatz kommen, daß sie bei einer mißbräuchlichen Applikation der Darreichungsform eine intensive negative Wirkung beim Mißbraucher hervorrufen. Dies gelingt vorzugsweise durch eine räumliche Trennung zumindest des Wirkstoffes bzw. der Wirkstoffe von den Komponenten (c) und/oder (d), wobei bevorzugt der Wirkstoff bzw. die Wirkstoffe in wenigstens einer Untereinheit (A) und die Komponenten (c) und/oder (d) in wenigstens einer Untereinheit (B) vorliegen, und wobei die Komponenten (c) und (d) bei bestimmungsgemäßer Applikation der Darreichungsform im Körper nicht ihre Wirkung entfalten.

Sofern die erfindungsgemäße Darreichungsform beide Komponenten (c) und (d) aufweist, können diese jeweils in derselben oder in verschiedenen Untereinheiten (B) vorliegen. Vorzugsweise liegen, sofern vorhanden, beide Komponenten (c) und (d) in ein- und derselben Untereinheit (B) vor.

Untereinheiten im Sinne der vorliegenden Erfindung sind feste Formulierungen, die jeweils neben üblichen, dem Fachmann bekannten Hilfsstoffen nur den (die) Wirkstoff(e) und gegebenenfalls wenigstens eine der gegebenenfalls vorhandenen Komponenten (a) und/oder (b) oder nur den (die) Antagonist(en) und/oder das Emetikum (die Emetika) und gegebenenfalls wenigstens eine der gegebenenfalls vorhandenen Komponenten (a) und/oder (b) enthalten.

Ein wesentlicher Vorteil der getrennten Formulierung der Wirkstoffe von den Komponenten (c) und (d) in Untereinheiten (A) und (B) der erfindungsgemäßen Darreichungsform besteht darin, daß bei ihrer bestimmungsgemäßen Applikation die Komponenten (c) und/oder (d) im Körper praktisch nicht freigesetzt werden, nur in so geringen Mengen freigesetzt werden, daß sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist. Vorzugsweise werden die Komponenten (c) und/oder (d) bei bestimmungsgemäßer Applikation der Darreichungsform im Körper des Patienten praktisch nicht freigesetzt. Der Fachmann versteht, daß diese vorstehend genannten Bedingungen in Abhängigkeit von den jeweils eingesetzten Komponenten (c) und (d) sowie der Formulierung der Untereinheiten bzw. der Darreichungsform variieren können. Die für die jeweilige Darreichungsform optimale Formulierung kann durch einfache Vorversuche ermittelt werden.

Wird eine entsprechende erfindungsgemäße Darreichungsform, welche die Komponenten (c) und/oder (d) in Untereinheiten (B) aufweist, zum Zwecke der mißbräuchlichen Einnahme des Wirkstoffes manipuliert, z.B. durch zermörsern und ggf. extrahieren des so erhaltenen Pulvers mit einem geeigneten Extraktionsmittel, wird neben dem Wirkstoff und gegebenenfalls (a) und/oder (b) auch die jeweilige Komponente (c) und/oder (d) in einer Form erhalten, in der sie von dem Wirkstoff nicht auf einfache Weise zu separieren ist, so daß sie bei der Applikation der manipulierten Darreichungsform, insbesondere bei oraler und/oder parenteraler Verabreichung, ihre Wirkung im Körper entfaltet und gegebenenfalls zusätzlich eine der Komponente (c) und/oder (d) entsprechende negative Wirkung beim Mißbraucher hervorruft und so den Mißbrauch der Darreichungsform verhindert.

Die Formulierung einer erfindungsgemäßen Darreichungsform, in der eine räumliche Trennung des Wirkstoffes bzw. der Wirkstoffe von den Komponenten (c) und (d), vorzugsweise durch Formulierung in verschiedenen Untereinheiten erfolgt ist, kann in vielfältiger Art und Weise erfolgen, wobei die entsprechenden Untereinheiten in der erfindungsgemäßen Darreichungsform jeweils in beliebiger räumlicher Anordnung zueinander vorliegen können, sofern die vorstehend genannten Bedingungen für die Freisetzung der Komponenten (c) und/oder (d) erfüllt sind.

Der Fachmann versteht, daß die ggf. vorliegenden Komponente(n) (a) und/oder (b) bevorzugt sowohl in den jeweiligen Untereinheiten (A) und (B) als auch in Form von eigenständigen, den Untereinheiten (A) und (B) entsprechenden Untereinheiten in der erfindungsgemäßen Darreichungsform formuliert werden können, so lange die Sicherung der Darreichungsform gegen den Mißbrauch wie auch die Wirkstofffreisetzung bei bestimmungsgemäßer Applikation durch die Art der Formulierung nicht beeinträchtig werden.

In einer bevorzugten Ausführungsform der erfindungsgemäßen Darreichungsform liegen die Untereinheiten (A) und (B) in multipartikulärer Form vor, wobei Mikrotabletten, Mikrokapseln, Mikropellets, Granulaten, Sphäroiden, Perlen oder Pellets bevorzugt sind und sowohl für die Untereinheit (A) als auch (B) dieselbe Form, d.h. Gestaltung gewählt wird, damit keine Separierung der Untereinheiten (A) von (B) durch mechanische Auslese möglich ist. Die multipartikulären Formen weisen bevorzugt eine Größe im Bereich von 0,1 bis 3 mm, vorzugsweise 0,5 bis 2 mm auf.

Die Untereinheiten (A) und (B) in multpartikulärer Form können auch bevorzugt in eine Kapsel abgefüllt, in einer Flüssigkeit oder einem Gel suspendiert oder zu einer Tablette verpreßt werden, wobei die jeweiligen Endformulierungen dergestalt erfolgen, daß die Untereinheiten (A) und (B) auch in der resultierenden Darreichungsform erhalten bleiben.

Die jeweiligen multipartikulären Untereinheiten (A) bzw (B) mit identischer Formgebung sollten auch nicht visuell voneinander unterscheidbar sein, damit sie vom Mißbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z.B. die Retardierung eines oder mehrerer Wirkstoffe oder eine magensaftresistente Ausrüstung der jeweiligen Untereinheiten.

In einer weiteren bevorzugten Ausführungsform der vorliegenden Erfindung sind die Untereinheiten (A) und (B) jeweils schichtförmig zueinander angeordnet.

Bevorzugt sind hierfür die schichtförmigen Untereinheiten (A) und (B) in der erfindungsgemäßen Darreichungsform vertikal oder horizontal zueinander angeordnet, wobei jeweils auch eine oder mehrere schichtförmige Untereinheiten (A) und eine oder mehrere schichtförmige Untereinheiten (B) in der Darreichungsform vorliegen können, so daß neben den bevorzugten Schichtenfolgen (A)-(B) bzw. (A)-(B)-(A) beliebige andere Schichtenfolgen in Betracht kommen, ggf. in Kombination mit Schichten enthaltend die Komponenten (a) und/oder (b).

Ebenfalls bevorzugt ist eine erfindungsgemäße Darreichungsform, in der die Untereinheit (B) einen Kern bildet, der von der Untereinheit (A) vollständig umhüllt wird, wobei zwischen diesen Schichten eine ggf. quellbare Trennschicht (C) vorhanden sein kann. Ein entsprechender Aufbau eignet sich bevorzugt auch für die vorstehend genannten multipartikulären Formen, wobei dann beide Untereinheiten (A) und (B) sowie eine ggf. vorhandene Trennschicht (C) in ein- und derselben multipartikulären Form formuliert sind.

In einer weiteren bevorzugten Ausführungsform der erfindungsgemäßen Darreichungsform bildet die Untereinheit (A) einen Kern, der von der Untereinheit (B) umhüllt wird, wobei letztere wenigstens einen Kanal aufweist, der von dem Kern an die Oberfläche der Darreichungsform führt.

Zwischen einer Schicht der Untereinheit (A) und einer Schicht der Untereinheit (B) kann die erfindungsgemäße Darreichungsform jeweils eine oder mehrere, vorzugsweise eine, ggf. quellbare Trennschicht (C) zur räumlichen Trennung der Untereinheit (A) von (B) aufweisen.

Sofern die erfindungsgemäße Darreichungsform die schichtförmigen Untereinheiten (A) und (B) sowie eine ggf. vorhandene Trennschicht (C) in einer zumindest teilweise vertikalen oder horizontalen Anordnung aufweist, liegt sie bevorzugt in Form einer Tablette, eines Coextrudats oder Laminats vor.

Hierbei kann in einer besonders bevorzugten Ausführungsform die freie Oberfläche der Untereinheit (B) vollständig und ggf. zumindest ein Teil der freien Oberfläche der Untereinheit(en) (A) und ggf. zumindest ein Teil der freien Oberfläche der ggf. vorhandenen Trennschicht(en) (C) mit wenigstens einer die Freisetzung des der Komponente (c) oder (d) verhindernden Barriereschicht (D) überzogen sein.

Ebenfalls besonders bevorzugt ist eine Ausführungsform der erfindungsgemäßen Darreichungsform, die eine vertikale oder horizontale Anordnung der Schichten der Untereinheiten (A) und (B) und wenigstens eine dazwischen angeordnete Push-Schicht (p) sowie ggf. eine Trennschicht (C) aufweist, in der sämtliche freie Oberflächen des aus den Untereinheiten (A) und (B), der Push-Schicht und der ggf. vorhandenen Trennschicht (C) bestehenden Schichtaufbaus mit einem semipermeablen Überzug (E) ausgerüstet sind, der für ein Freisetzungsmedium, d.h. üblicherweise eine physiologische Flüssigkeit, durchlässig, für den Wirkstoff und für die Komponente (c) und/oder (d) im wesentlichen undurchlässig ist, und wobei dieser Überzug (E) im Bereich der Untereinheit (A) wenigstens eine Öffnung zur Freisetzung des Wirkstoffes aufweist.

Eine entsprechende Darreichungsform ist dem Fachmann beispielsweise unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt. Die entsprechenden Beschreibungen werden hiermit als Referenz eingeführt und gelten als Teil der Offenbarung.

In einer weiteren bevorzugten Ausführungsform hat die Untereinheit (A) der erfindungsgemäßen Darreichungsform die Form einer Tablette, deren Steg und ggf. eine der beiden Grundflächen mit einer die Komponente (c) und/oder (d) enthaltenden Barriereschicht (B) bedeckt ist.

Der Fachmann versteht, daß die bei der Formulierung der erfindungsgemäßen Darreichungsform jeweils zum Einsatz kommenden Hilfsstoffe der Untereinheit(en) (A) bzw. (B) sowie ggf. der vorhandenen Trennschicht(en) (C) und/oder der Barriereschicht(en) (D) in Abhängigkeit von deren Anordnung in der erfindungsgemäßen Darreichungsform, der Applikationsart sowie in Abhängigkeit von dem jeweiligen Wirkstoff der ggf. vorhandenen Komponenten (a) und/oder (b) und der Komponente (c) und/oder (d) variieren. Die Materialien, die über die jeweils erforderlichen Eigenschaften verfügen sind, dem Fachmann an sich bekannt.

Sofern die Freisetzung der Komponente (c) und/oder (d) aus der Untereinheit (B) der erfindungsgemäßen Darreichungsform mit Hilfe einer Umhüllung, vorzugsweise einer Barriereschicht, verhindert wird, kann die Untereinheit aus üblichen, dem Fachmann bekannten Materialien bestehen.

Ist eine entsprechende Barriereschicht (D) zur Verhinderung der Freisetzung der Komponente (c) und/oder (d) nicht vorgesehen, sind die Materialien der Untereinheiten so zu wählen, daß eine Freisetzung der jeweiligen Komponente (c) und/oder (d) aus der Untereinheit (B) praktisch ausgeschlossen ist.
Bevorzugt können hierzu die nachstehend aufgeführten Materialien zum Einsatz kommen, die auch für den Aufbau der Barriereschicht geeignet sind.

Bevorzugte Materialien sind solche, die ausgewählt sind aus der Gruppe bestehend aus Alkylcellulosen, Hydroxyalkylcellulosen, Glucanen, Skleroglucanen, Mannanen, Xanthanen, Copolymeren aus Poly[bis(p-carboxyphenoxy)propan und Sebacinsäure, vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane sowie Polyurethane und deren Copolymeren.

Besonders geignete Materialien können ausgewählt werden aus der Gruppe bestehend aus Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe bestehend aus Copolymeren aus Butylmethacrylat und Isobutylmethacrylat, Copolymeren aus Methylvinylether und Maleinsäure mit erhöhtem Molekulargewicht, Copolymeren aus Methylvinylether und Maleinsäuremonoethylester, Copolymeren aus Methylvinylether und Maleinsäureanhydrid sowie Copolymeren aus Vinylalkohol und Vinylacetat.

Weitere, zur Formulierung der Barriereschicht besonders geeignete Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1). Die entsprechenden Beschreibungen werden hiermit als Referenz eingeführt und gelten als Teil der Offenbarung.

Ggf. können die vorstehend genannten Materialien mit weiteren üblichen, dem Fachmann bekannten Hilfsstoffen, vorzugsweise ausgewählt aus der Gruppe bestehend aus Glycerylmonostearat, halbsynthetischen Triglyceridderivaten, halbsynthetischen Glyceriden, hydriertem Rizinusöl, Glycerylpalmitostearat, Glycerylbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierten Triglyceriden, Glyceriden, Polyoxyalkylenglykolen und deren Derivate abgemischt werden.

Sofern die erfindungsgemäße Darreichungsform eine Trennschicht (D) aufweist, kann diese, ebenso wie die nicht umhüllte Untereinheit (B) vorzugsweise aus den vorstehend, für die Barriereschicht beschriebenen Materialien bestehen. Der Fachmann versteht, daß auch über die Dicke der Trennschicht die Freisetzung des Wirkstoffes bzw. der Komponente (c) und/oder (d) aus der jeweiligen Untereinheit gesteuert werden kann.

Die erfindungsgemäße Darreichungsform kann einen oder mehrere Wirkstoffe zumindest teilweise in retardierter Form aufweisen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge. Die Wirkstoffabgabe muß aber so gesteuert sein, daß die vorstehend genannten Bedingungen jeweils erfüllt sind, z.B. das bei bestimmungsgemäßer Applikation der Darreichungsform der Wirkstoff bzw. die Wirkstoffe praktisch komplett freigesetzt wird, bevor die Komponente (c) und/oder (d) eine beeinträchtigende Wirkung entfalten kann.

Sofern die erfindungsgemäße Darreichungsform zur oralen Applikation vorgesehen ist, kann sie bevorzugt auch einen magensaftresistenten Überzug aufweisen, der sich in Abhängigkeit vom pH-Wert der Freisetzungsumgebung auflöst.
Durch diesen Überzug kann erreicht werden, daß die erfindungsgemäße Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf.

Entsprechende Materialien und Verfahren zur Retardierung von Wirkstoffen sowie zum Aufbringen magensaftresistenter Überzüge sind dem Fachmann beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt. Die entsprechende Literaturbeschreibung wird hiermit als Referenz eingeführt und gilt als Teil der Offenbarung.

Die erfindungsgemäßen Darreichungsformen haben den Vorteil, daß sie durch eine beliebige Kombination von zwei oder mehr der Komponenten (a)-(d) gegen jegliche Art von Mißbrauch, vorzugsweise gegen oralen, nasalen und parenteralen Miißbruach, geschützt sind, ohne daß bei bestimmungsgemäßer Applikation eine Beeinträchtigung des zu therapierenden Patienten oder eine Verminderung der Wirksamkeit des jeweiligen Wirkstoffes zu befürchten ist. Sie lassen sich einfach und vergleichsweise kostengünstig produzieren.

Im folgenden wird die Erfindung anhand von Beispielen erläutert, Diese Erläuterungen sind lediglich beispielhaft und schränken den allgemeinen Erfindungsgedanken nicht ein.

### Beispiele:

### Beispiel 1

Matrixtabletten mit folgender Zusammensetzung pro Tablette

| | |
|---|---|
| (-)-(1 R,2R)-3-(3-Dimethylamino-1-ethyl-2-methyl-propyl)phenol-Hydrochlorid | 100 mg |
| Hydroxypropylmethylcellulose (Metolose 90 SH 100.000 von Fa. Fa. Shinetsu), 100.000 mPa·s | 70 mg |
| Xanthan, NF | 10 mg |
| Mikrokristalline Cellulose (Avicel PH 102 von Fa. FA. FMC) | 113 mg |
| Cayennepfeffer | 10 mg |
| Hochdisperses Siliciumdioxid | 4 mg |
| Magnesiumstearat | 3 mg |
| Gesamtmenge | 310 mg |

wurden in einer Ansatzgröße von 1000 Tabletten in folgender Weise hergestellt: Alle Bestandteile wurden eingewogen und auf einer Siebmaschine Quadro Comil U10 unter Verwendung einer Siebgröße von 0,813 mm gesiebt, in einem Containermischer (Bohle LM 40) 15 min ± 15 s bei einer Drehzahl von 20 ± 1 U/min gemischt und auf einer Korsch EK0 Exzenterpresse zu drageegewölbten Tabletten mit einem Durchmesser von 10 mm, einem Wölbungsradius von 8 mm und einem mittleren Tablettengewicht von 310 mg gepreßt.

Eine der Tabletten wird gemörsert und mit 10 ml Wasser geschüttelt. Es bildet sich eine viskose, trübe Suspension. Nach Absetzen der groben, festen Bestandteile der Suspension wird diese in eine Spritze mit einer Nadel mit 0,9 mm aufgezogen, wobei das Aufziehen aufgrund der Viskosität erschwert ist. Die aufgezogene Extraktionsflüssigkeit wird in 37° C warmes Wasser gespritzt, wobei deutlich Fäden mit dem Durchmesser der Nadel extrudiert werden, die sich nicht mit dem Wasser mischen. Unter Rühren werden die Fäden geteilt, aber nicht gelöst; nach einigen Minuten sind immer noch die Bruchstücke der Fäden mit dem bloßen Auge erkennbar. Bei Injektion eines derartigen Extraktes in Blutgefäße käme es zu Verstopfungen in den Gefäßen.

Eine gemörserte Tablette wurde zu einer 10 cm langen Linie ausgezogen und durch ein Röhrchen in die Nase eingesaugt. Bereits nach dem ersten cm wurde die Nase derart gereizt, daß das dringende Bedürfnis des Entfernen des Pulvers durch Niesen erzeugt wurde, eine weitere nasale Zufuhr des restlichen Pulvers ist nicht mehr möglich. Nach dem Abniesen klang die Nasenreizung ab, und nach ca. 10 min war der Reiz weitgehend verschwunden. Wiederholungsbedürfnis kommt nicht auf.

### Beispiel 2

Matrixtabletten mit folgender Zusammensetzung pro Tablette

| | |
|---|---|
| (-)-(1 R,2R)-3-(3-Dimethylamino-1-ethyl-2-methyl-propyl)phenol-Hydrochlorid | 100 mg |
| Hydroxypropylmethylcellulose (Metolose 90 SH 100.000 von Fa. Fa. Shinetsu), 100.000 mPa·s | 40 mg |
| Xanthan, NF | 40 mg |
| Mikrokristalline Cellulose (Avicel PH 102 von Fa. FA. FMC) | 113 mg |
| Cayennepfeffer | 10 mg |
| Hochdisperses Siliciumdioxid | 4 mg |
| Magnesiumstearat | 3 mg |
| Gesamtmenge | 310 mg |

wurden wie in Beispiel 1 angegeben hergestellt.

Eine der Tabletten wurden gemörsert und mit 10 ml Wasser geschüttelt. Es bildete sich eine viskose, trübe Suspension, deren Viskosität höher als in Beispiel 1 war; auch wurden mehr Luftblasen eingeschlossen. Nach Absetzen der groben, festen Bestandteile der Suspension wird diese in eine Spritze mit einer Nadel mit 0,9 mm aufgezogen, wobei das Aufziehen aufgrund der Viskosität sehr erschwert ist. Die aufgezogene Extraktionsflüssigkeit wird in 37° C warmes Wasser gespritzt, wobei deutlich Fäden mit dem Durchmesser der Nadel extrudiert werden, die sich nicht mit dem Wasser mischen. Unter Rühren werden die Fäden geteilt, aber nicht gelöst; nach einigen Minuten sind immer noch die Bruchstücke der Fäden mit dem bloßen Auge erkennbar. Bei Injektion eines derartigen Extraktes in Blutgefäße käme es zu Verstopfungen in den Gefäßen.

Eine gemörserte Tablette wurde zu einer 10 cm langen Linie ausgezogen und durch ein Röhrchen in die Nase eingesaugt. Bereits nach dem ersten cm wurde die Nase derart gereizt, daß das dringende Bedürfnis des Entfernen des Pulvers durch Niesen erzeugt wurde, eine weitere nasale Zufuhr des restlichen Pulvers ist nicht mehr möglich. Nach dem Abniesen klang die Nasenreizung ab, und nach ca. 10 min. war der Reiz weitgehend verschwunden. Wiederholungsbedürfnis kommt nicht auf.

### Beispiel 3

Matrixtabletten mit folgender Zusammensetzung pro Tablette

| | |
|---|---|
| (-)-(1R,2R)-3-(3-Dimethylamino-1-ethyl-2-methyl-propyl)phenol-Hydrochlorid | 100 mg |
| Xanthan, NF | 80 mg |
| Mikrokristalline Cellulose (Avicel PH 102 von Fa. FA. FMC) | 113 mg |
| Cayennepfeffer | 10 mg |
| Hochdisperses Siliciumdioxid | 4 mg |
| Magnesiumstearat | 3 mg |
| Gesamtmenge | 310 mg |

wurden wie in Beispiel 1 angegeben hergestellt.

Eine der Tabletten wurde gemörsert und mit 10 ml Wasser geschüttelt. Es bildete sich eine viskose, trübe Suspension, deren Viskosität höher als in Beispiel 1 war; auch wurden noch mehr Luftblasen eingeschlossen. Nach Absetzen der groben, festen Bestandteile der Suspension wird diese in eine Spritze mit einer Nadel mit 0,9 mm aufgezogen, wobei das Aufziehen aufgrund der Viskosität sehr erschwert ist Die aufgezogene Extraktionsflüssigkeit wird in 37° C warmes Wasser gespritzt, wobei deutlich Fäden mit dem Durchmesser der Nadel extrudiert werden, die sich nicht mit dem Wasser mischen. Unter Rühren werden die Fäden geteilt, aber nicht gelöst; nach einigen Minuten sind immer noch die Bruchstücke der Fäden mit dem bloßen Auge erkennbar. Bei Injektion eines derartigen Extraktes in Blutgefäße käme es zu Verstopfungen in den Gefäßen.

Eine gemörserte Tablette wurde zu einer 10 cm langen Linie ausgezogen und durch ein Röhrchen in die Nase eingesaugt. Bereits nach dem ersten cm wurde die Nase derart gereizt, daß das dringende Bedürfnis des Entfernen des Pulvers durch Niesen erzeugt wurde, eine weitere nasale Zufuhr des restlichen Pulvers ist nicht mehr möglich. Nach dem Abniesen klang die Nasenreizung ab, und nach ca. 10 min war der Reiz weitgehend verschwunden. Wiederholungsbedürfnis kommt nicht auf.

### Beispiele 4-7

Matrixtabletten mit folgender Zusammensetzung pro Tablette

| **Beispiel** | **4** | **5** | **6** | **7** |
|---|---|---|---|---|
| (-)-(1 R,2R)-3-(3-Dimethylamino-1-ethyl-2-methyl-propyl)phenol-Hydrochlorid | 100 mg | 100 mg | 100 mg | 100 mg |
| Hydroxypropylmethylcellulose (Metolose 90 SH 100.000 von Fa. Fa. Shinetsu), 100.000 mPa·s | 80 mg | 80 mg | 80 mg | 80 mg |
| Carboxymethylcellulose (Tylose C300) | 10 mg | | | |
| Carboxymethylcellulose (Tylose C600) | | 10 mg | | |
| Hydroxyethylcellulose (Tylose H300) | | | 10 mg | |
| Hydroxyethylcellulose (Tylose H4000) | | | | 10 mg |
| Mikrokristalline Cellulose (Avicel PH 102 von Fa. FA. FMC) | 113 mg | 113 mg | 113 mg | 113 mg |
| Cayennepfeffer | 10 mg | 10 mg | 10 mg | 10 mg |
| Hochdisperses Siliciumdioxid | 4 mg | 4 mg | 4 mg | 4 mg |
| Magnesiumstearat | 3 mg | 3 mg | 3 mg | 3 mg |
| Gesamtmenge | 320 mg | 320 mg | 320 mg | 320 mg |

wurden wie in Beispiel 1 angegeben hergestellt.

Die Tabletten wurden gemörsert und mit 10ml Wasser geschüttelt. Es bildete sich eine viskose, trübe Suspension; auch wurden Luftblasen eingeschlossen. Nach Absetzen der groben, festen Bestandteile der Suspension wird diese in eine Spritze mit einer Nadel mit 0,9 mm aufgezogen, wobei das Aufziehen aufgrund der Viskosität sehr erschwert ist. Die aufgezogene Extraktionsflüssigkeit wird in 37° C warmes Wasser gespritzt, wobei deutlich Fäden mit dem Durchmesser der Nadel extrudiert werden, die sich nicht mit dem Wasser mischen. Unter Rühren werden die Fäden geteilt, aber nicht gelöst; nach einigen Minuten sind immer noch die Bruchstücke der Fäden mit dem bloßen Auge erkennbar. Bei Injektion eines derartigen Extraktes in Blutgefäße käme es zu Verstopfungen in den Gefäßen.

Eine gemörserte Tablette wurden jeweils zu einer 10 cm langen Linie ausgezogen und durch ein Röhrchen in die Nase eingesaugt. Bereits nach dem ersten cm wurde die Nase derart gereizt, daß das dringende Bedürfnis des Entfernen des Pulvers durch Niesen erzeugt wurde, eine weitere nasale Zufuhr des restlichen Pulvers ist nicht mehr möglich. Nach dem Abniesen klang die Nasenreizung ab, und nach ca. 10 min war der Reiz weitgehend verschwunden. Wiederholungsbedürfnis kommt nicht auf.

### Beispiele 8-13

Matrixtabletten mit folgender Zusammensetzung pro Tablette

| **Beispiel** | **8** | **9** | **10** | **11** | **12** | **13** |
|---|---|---|---|---|---|---|
| Morphinsulfat Pentahydrat | 60 mg | 60 mg | 60 mg | 60 mg | 60 mg | 60 mg |
| Hydroxypropylmethylcellu lose (Metolose 90 SH 15.000 von Fa. Fa. Shinetsu), 15.000 mPa·s | 60 mg | 60 mg | 60 mg | 60 mg | 60 mg | 60 mg |
| Xanthan, NF | 10 mg | 30 mg | | | | |
| Carboxymethylcellulose (Tylose C300) | | | 10 mg | | | |
| Carboxymethylcellulose (Tylose C600) | | | | 10 mg | | |
| Hydroxyethylcellulose (Tylose H300) | | | | | 10 mg | |
| Hydroxyethylcellulose (Tylose H4000) | | | | | | 10 mg |
| Mikrokristalline Cellulose (Avicel PH 102 von Fa. FA. FMC) | 112,9 mg | 112,9 mg | 112,9 mg | 112,95 mg | 112,95 mg | 112,95 mg |
| Capsaicin, mikronisiert | 0,1 mg | 0,1 mg | 0,1 mg | 0,05 mg | 0,05 mg | 0,05 mg |
| Hochdisperses Siliciumdioxid | 4 mg | 4 mg | 4 mg | 4 mg | 4 mg | 4 mg |
| Magnesiumstearat | 3 mg | 3 mg | 3 mg | 3 mg | 3 mg | 3 mg |

wurden wie in Beispiel 1 angegeben hergestellt.

Eine Tablette wurde gemörsert und mit 10ml Wasser geschüttelt. Es bildete sich eine viskose, trübe Suspension; auch wurden Luftblasen eingeschlossen. Nach Absetzen der groben, festen Bestandteile der Suspension wird diese in eine Spritze mit einer Nadel mit 0,9 mm aufgezogen, wobei das Aufziehen aufgrund der Viskosität sehr erschwert ist. Die aufgezogene Extraktionsflüssigkeit wird in 37° C warmes Wasser gespritzt, wobei deutlich Fäden mit dem Durchmesser der Nadel extrudiert werden, die sich nicht mit dem Wasser mischen. Unter Rühren werden die Fäden geteilt, aber nicht gelöst; nach einigen Minuten sind immer noch die Bruchstücke der Fäden mit dem bloßen Auge erkennbar. Bei Injektion eines derartigen Extraktes in Blutgefäße käme es zu Verstopfungen in den Gefäßen.

Eine gemörserte Tablette wurden jeweils zu einer 10 cm langen Linie ausgezogen und durch ein Röhrchen in die Nase eingesaugt. Bereits nach dem ersten cm wurde die Nase derart gereizt, daß das dringende Bedürfnis des Entfernen des Pulvers durch Niesen erzeugt wurde, eine weitere nasale Zufuhr des restlichen Pulvers ist nicht mehr möglich. Nach dem Abniesen klang die Nasenreizung ab, und nach ca. 10 min war der Reiz weitgehend verschwunden. Wiederholungsbedürfnis kommt nicht auf.

### Beispiele 14-18

Kapseln mit folgender Zusammensetzung der einfachen Pulvermischung pro Kapsel (Kapselgröße 4)

| **Beispiel** | **14** | **15** | **16** | **17** | **18** |
|---|---|---|---|---|---|
| Morphinsulfat Pentahydrat | 20 mg | 20 mg | 20 mg | 20 mg | 20 mg |
| Xanthan, NF | 10 mg | | | | |
| Carboxymethylcellulose (Tylose C300) | | 10 mg | | | |
| Carboxymethylcellulose (Tylose C600) | | | 10 mg | | |
| Hydroxyethylcellulose (Tylose H300) | | | | 10 mg | |
| Hydroxyethylcellulose (Tylose H4000) | | | | | 10 mg |
| Mikrokristalline Cellulose (Avicel PH 102 von Fa. FA. FMC) | 63 mg | 63 mg | 63 mg | 63 mg | 63 mg |
| Cayennepfeffer | 5 mg | 5 mg | 5 mg | 5 mg | 5 mg |
| Hochdisperses Siliciumdioxid | 1 mg | 1 mg | 1 mg | 1 mg | 1 mg |
| Magnesiumstearat | 1 mg | 1 mg | 1 mg | 1 mg | 1 mg |

Das Pulver aus den Kapseln wurde gemörsert und mit 10ml Wasser geschüttelt. Es bildete sich eine viskose, trübe Suspension; auch wurden Luftblasen eingeschlossen. Nach Absetzen der groben, festen Bestandteile der Suspension wird diese in eine Spritze mit einer Nadel mit 0,9 mm aufgezogen, wobei das Aufziehen aufgrund der Viskosität sehr erschwert ist. Die aufgezogene Extraktionsflüssigkeit wird in 37° C warmes Wasser gespritzt, wobei deutlich Fäden mit dem Durchmesser der Nadel extrudiert werden, die sich nicht mit dem Wasser mischen. Unter Rühren werden die Fäden geteilt, aber nicht gelöst; nach einigen Minuten sind immer noch die Bruchstücke der Fäden mit dem bloßen Auge erkennbar. Bei Injektion eines derartigen Extraktes in Blutgefäße käme es zu Verstopfungen in den Gefäßen.

Das gemörserte Pulver wurden jeweils zu einer 10 cm langen Linie ausgezogen und durch ein Röhrchen in die Nase eingesaugt. Bereits nach dem ersten cm wurde die Nase derart gereizt, daß das dringende Bedürfnis des Entfernen des Pulvers durch Niesen erzeugt wurde, eine weitere nasale Zufuhr des restlichen Pulvers ist nicht mehr möglich. Nach dem Abniesen klang die Nasenreizung ab, und nach ca. 10 min war der Reiz weitgehend verschwunden. Wiederholungsbedürfnis kommt nicht auf.

Die folgenden Mengenangaben beziehen sich jeweils auf die Zusammensetzung einer Darreichungsform. Eine Charge eines Herstellgangs besteht aus 1000 solcher Darreichungsformen.

### Beispiel 19

### Manteltabletten

**Kern**

| | |
|---|---|
| Emetin | 50 mg |
| Hydriertes Rizinusöl (Cutina HR) | 50 mg |

Emetin und fein gepulvertes Hydriertes Rizinusöl werden gemischt und auf einer Tablettenpresse zu runden, bikonvexen Tabletten mit 6,5 mm Durchmesser verpreßt.

**Mantel**

| | |
|---|---|
| Morphinsulfat Pentahydrat | 60 mg |
| Methylhydroxypropylcellulose 100 000 mPas (Metolose 90 SH 100 000, ShinEtsu) | 100 mg |
| Mikrokristalline Cellulose (Avicel PH 102) | 155 mg |
| Lactose Monohydrat | 165 mg |
| Cayennepfeffer | 10 mg |
| Magnesiumstearat | 5 mg |
| Kolloidales Siliciumdioxid | 5 mg |

Alle Mantelbestandteile werden gemischt, in einer Tablettenpresse mit Werkzeug für 13 mm bikonvexe Tabletten werden ca. 250 mg der Mischung in die Tablettenmatrize gefüllt, der 6,5 mm Kern zentriert eingelegt, die restlichen 250 mg der Mantelmischung zugefüllt und der Mantel um den Kern gepreßt.

### Beispiel 20

### Manteltabletten

**Kern**

| | |
|---|---|
| Emetin | 50 mg |
| Hydriertes Rizinusöl (Cutina HR) | 50 mg |

Emetin und fein gepulvertes Hydriertes Rizinusöl werden gemischt und auf einer Tablettenpresse zu runden, bikonvexen Tabletten mit 6,5 mm Durchmesser verpreßt.

**Mantel**

| | |
|---|---|
| Oxycodon-Hydrochlorid | 30 mg |
| Srühgetrocknete Lactose | 290 mg |
| Eudragit RSPM | 70 mg |
| Stearylakohol | 115 mg |
| Cayennepfeffer | 10 mg |
| Magnesiumstearat | 5 mg |
| Talkum | 10 mg |

Oxycodon-Hydrochlorid, sprühgetrocknete Lactose und Eudragit RSPM werden in einem geeigneten Mischer ca. 5 min innig miteinander vermischt. Während des Mischens wird die Mischung mit einer solchen Menge Gereinigtem Wasser granuliert, daß sich eine feuchte granulierte Masse bildet. Das so erhaltene Granulat wird in der Wirbelschicht bei 60°C getrocknet und durch ein 2,5 mm Sieb gesiebt. Anschließend wird das Granulat erneut wie vor beschrieben getrocknet und durch ein 1,5 mm Sieb gegesiebt. Der Stearylakohol wird bei 60-70°C geschmolzen und in einem Mischer zu dem Granulat gegeben. Nach dem Abkühlen wird die Masse zusammen mit Cayennepfeffer, Magnesiumstearat und Talkum durch ein 1,5 mm Sieb gesiebt. Vom so erhaltenen Granulat wird in einer Tablettenpresse mit Werkzeug für 13 mm bikonvexe Tabletten ca. 265 mg der Mischung in die Tablettenmatrize gefüllt, der 6,5 mm Kern zentriert eingelegt, die restlichen 265 mg der Mantelmischung zugefüllt und der Mantel um den Kern gepreßt.

### Beispiel 21

### Manteltabletten

**Kern**

| | |
|---|---|
| Naltrexonhydrochlorid | 50 mg |
| Sprühgetrocknete Lactose | 46 mg |
| Magnesiumstearat | 2 mg |
| Kolloidales Siliciumdioxid | 2 mg |

Alle Bestandteile werden gemischt und auf einer Tablettenpresse zu runden, bikonvexen Tabletten mit 6,5 mm Durchmesser verpreßt

**Überzug auf Kern**

| | |
|---|---|
| Celluloseacetat mit 39,8% Acetat | 9,5 mg |
| Macrogol 3350 | 0,5 mg |

Die Überzugsbestandteile werden in einem Aceton-Wasser Gemisch (95:5 Gewichtsteile) gelöst und auf die Kerne gesprüht

**Mantel**

| | |
|---|---|
| Morphinsulfat Pentahydrat | 60 mg |
| Methylhydroxypropylcellulose 100 000 mPas (Metolose 90 SH 100 000, ShinEtsu) | 100 mg |
| Mikrokristalline Cellulose (Avicel PH 102) | 165 mg |
| Lactose Monohydrat | 155 mg |
| Cayennepfeffer | 10 mg |
| Magnesiumstearat | 5 mg |
| Kolloidales Siliclumdioxid | 5 mg |

Alle Mantelbestandteile werden gemischt, in einer Tablettenpresse mit Werkzeug für 13 mm bikonvexe Tabletten wurden ca. 250 mg der Mischung in die Tablettenmatrize gefüllt, der mit Celluloseacetat überzogene Kern zentriert eingelegt, die restlichen 250 mg der Mantelmischung zugefüllt und der Mantel um den Kern gepreßt.

### Beispiel 22

### Multipartikuläre Form

**Emetika Pellets**

| | |
|---|---|
| Emetin | 50 mg |
| Lactose | 15 mg |
| Mikrokristalline Cellulose PH101 | 30 mg |
| Niedrigsubstituierte Hydroxypropylcellulose (LH31, Shin-Etsu) | 5 mg |

Alle Bestandteile werden in einem geeigneten Mischer ca. 5 min innig miteinander vermischt. Während des Mischens wird die Mischung mit einer solchen Menge Gereinigtem Wasser granuliert, daß sich eine feuchte granulierte Masse bildet. Das so erhaltene Granulat wird in einem Nica-Extruder durch eine Matrize mit Extrusionsöffnungen von 1 mm extrudiert, 5 min auf einem Spheronizer gerundet, in der Wirbelschicht bei 60°C getrocknet und mittels eines 1,5 mm und eines 0,5 mm Siebs klassiert.

**Überzug auf Emetika Pellets**

| | |
|---|---|
| Celluloseacetat mit 39,8 % Acetat | 9,5 mg |
| Macrogol 3350 | 0,5 mg |
| Titandioxid | 0,5 mg |

Mengenangaben pro 100 mg Emetikapellets

Celluloseacetat und Macrogol werden in einem Aceton-Wasser Gemisch (95:5 Gewichtsteile) 3,8% ige Lösung gelöst, Titandioxid wird in der Mischung dispergiert und die Kerne in einer Wirbelschichtanlage mit der Suspension besprüht bis die Masse der überzogenen Pellets 110% des Gewichts der eingesetzten nicht überzogenen Pellets beträgt.

**Analgetica Pellets**

| | |
|---|---|
| Nonpareils 0,5 mm (Saccharose-Maisstärke Starter Pellets, Fa. Werner) | 50 mg |
| Morphinsulfat Pentahydrat | 60 mg |
| Povidon K 30 | 30 mg |
| Capsaicin | 0,1 mg |
| Talkum | 9,9 mg |

Morphinsulfat und Povidon werden in Gereinigtem Wasser gelöst und Talkum wird in der Lösung dispergiert. Capsaicin wird als 10%ige Lösung in Ethanol gelöst und die Lösung wird der Suspension zugegeben. Die Suspension wird in bei 60°C auf die Nonpareils aufgesprüht und getrocknet. Die Pellets werden mittels eines 1,5 mm und eines 0,5 mm Siebs klassiert.

**Überzug auf Analgetica Pellets**

| | |
|---|---|
| Ethylcellulose Dispersion (Aquacoat ECD 30, FMC Corporation) | 10,0 mg |
| Glycerolmonostearat | 2,0 mg |
| Talkum | 2,0 mg |
| Titandioxid | 1,0 mg |

Mengenangaben pro 150 mg Analgetica Pellets, Ethylcellulosegewichtsangabe als Trockenmasse aus der 30%igen Dispersion des Handelsprodukts.

Ethylcellulose Dispersion wird 1:0,5 mit Gereinigtem Wasser gemischt und das Glycerolmonostearat wird unter mindestens zwei stündigem Rühren eingearbeitet. Talkum und Titandioxid werden in 0,5 Teilen Wasser (Berechnungsbasis aus der 1:0,5 Mischung der Ethylcellulose Dispersion) dispergiert und mit der Ethylcellulosedispersion gemischt. Die Analgetica Pellets werden in einer Wirbelschichtanlage mit der Dispersion besprüht bis die Masse der überzogenen Pellets 110% des Gewichts der eingesetzten nicht überzogenen Pellets beträgt.

### Kapseln

Pro Kapsel werden jeweils 110 mg überzogene Emetika Pellets und 165 mg überzogene Analgetica Pellets gemischt und in Hartgelatinekapseln der Größe 1 abgefüllt.

### Beispiel 23

### Multipartikuläre Form

**Antagonisten Pellets**

| | |
|---|---|
| Naloxonhydrochlorid-Dihydrat | 20 mg |
| Lactose | 7 mg |
| Mikrokristalline Cellulose PH101 | 20 mg |
| Niedrigsubstituierte Hydroxypropylcellulose (LH31, Shin-Etsu) | 3 mg |

Alle Bestandteile werden in einem geeigneten Mischer ca. 5 min innig miteinander vermischt. Während des Mischens wird die Mischung mit einer solchen Menge Gereinigtem Wasser granuliert, daß sich eine feuchte granulierte Masse bildet. Das so erhaltene Granulat wird in einem Nica-Extruder durch eine Matrize mit Extrusionsöffnungen von 1 mm extrudiert, 5 min auf einem Spheronizer gerundet, in der Wirbelschicht bei 60°C getrocknet und mittels eines 1,5 mm und eines 0,5 mm Siebs klassiert.

**Überzug auf Antagonisten Pellets**

| | |
|---|---|
| Celluloseacetat mit 39,8 % Acetat | 9,5 mg |
| Macrogol 3350 | 0,5 mg |
| Titandioxid | 0,5 mg |

Mengenangaben pro 100 mg Emetikapellets

Celluloseacetat und Macrogol werden in einem Aceton-Wasser Gemisch (95:5 Gewichtsteile) 3,8% ige Lösung gelöst, Titandioxid wird in der Mischung dispergiert und die Kerne in einer Wirbelschichtanlage mit der Suspension besprüht bis die Masse der überzogenen Pellets 110% des Gewichts der eingesetzten nicht überzogenen Pellets beträgt.

**Analgetica Pellets**

| | |
|---|---|
| Nonpareils 0,5 mm (Saccharose-Maisstärke Starter Pellets, Fa. Werner) | 50 mg |
| Morphinsulfat Pentahydrat | 60 mg |
| Povidon K 30 | 30 mg |
| Cayennepfeffer | 5 mg |
| Talkum | 10 mg |

Morphinsulfat und Povidon werden in Gereinigtem Wasser gelöst und Cayennepfeffer und Talkum werden in der Lösung dispergiert. Die Suspension wird in bei 60°C auf die Nonpareils aufgesprüht und getrocknet. Die Pellets werden mittels eines 1,5 mm und eines 0,5 mm Siebs klassiert.

**Überzug auf Analgetica Pellets**

| | |
|---|---|
| Ethylcellulose Dispersion (Aquacoat ECD 30, FMC Corporation) | 10,0 mg |
| Glycerolmonostearat | 2,0 mg |
| Talkum | 2,0 mg |
| Titandioxid | 1,0 mg |

Mengenangaben pro 150 mg Analgetica Pellets, Ethylcellulosegewichtsangabe als Trockenmasse aus der 30%igen Dispersion des Handelsprodukts.

Ethylcellulose Dispersion wird 1:0,5 mit Gereinigtem Wasser gemischt und das Glycerolmonostearat wird unter mindestens zwei stündigem Rühren eingearbeitet. Talkum und Titandioxid werden in 0,5 Teilen Wasser (Berechnungsbasis aus der 1:0,5 Mischung der Ethylcellulose Dispersion) dispergiert und mit der Ethylcellulosedispersion gemischt. Die Analgetica Pellets werden in einer Wirbelschichtanlage mit der Dispersion besprüht bis die Masse der überzogenen Pellets 110% des Gewichts der eingesetzten nicht überzogenen Pellets beträgt.

### Kapseln

Pro Kapsel werden jeweils 55 mg überzogene Antagonisten Pellets und 170 mg überzogene Analgetica Pellets gemischt und in Hartgelatinekapseln der Größe 2 abgefüllt.

### Beispiel 24

### Manteltabletten

**Kern**

| | |
|---|---|
| Emetinhydrochlorid-Pentahydrat | 60 mg |
| Hydriertes Rizinusöl (Cutina HR) | 40 mg |

Emetinhydrochlorid-Pentahydrat und fein gepulvertes Hydriertes Rizinusöl werden gemischt und auf einer Tablettenpresse zu runden, bikonvexen Tabletten mit 6,5 mm Durchmesser verpreßt.

**Überzug auf Kern**

| | |
|---|---|
| Celluloseacetat mit 39,8 % Acetat | 9,5 mg |
| Macrogol 3350 | 0,5 mg |

Die Überzugsbestandteile werden in einem Aceton-Wasser Gemisch (95:5 Gewichtsteile) als 3,8% ige Lösung gelöst und auf die Kerne gesprüht.

**Mantel**

| | |
|---|---|
| Morphinsulfat Pentahydrat | 60 mg |
| Methylhydroxypropylcellulose 100 000 mPas (Metolose 90 SH 100 000, ShinEtsu) | 100 mg |
| Mikrokristalline Cellulose (Avicel PH 102) | 165 mg |
| Lactose Monohydrat | 164,9 mg |
| Capsaicin, mikronisiert | 0,1 mg |
| Magnesiumstearat | 5 mg |
| Kolloidales Siliciumdioxid | 5 mg |

Alle Mantelbestandteile werden gemischt, in einer Tablettenpresse mit Werkzeug für 13 mm bikonvexe Tabletten werden ca. 250 mg der Mischung in die Tablettenmatrize gefüllt, der mit Celluloseacetat überzogene 6,5 mm Kern zentriert eingelegt, die restlichen 250 mg der Mantelmischung zugefüllt und der Mantel um den Kern gepreßt.

### Beispiel 25

### Orales osmotisches therapeutisches System (OROS)

**Wirkstoffschicht**

| | |
|---|---|
| Morphinsulfat Pentahydrat | 125 mg |
| Macrogol 200 000 | 280 mg |
| Povidon (MG_{N} 40 000) | 26 mg |
| Cayennepfeffer | 15 mg |
| Magnesiumstearat | 4 mg |

Morphinsulfat und Macrogol werden in einem Planetenmischer trocken gemischt und anschließend unter langsamer Zugabe einer Lösung des Povidon in 115 mg Ethanol zu einer feuchten Masse angeteigt, die dann durch ein 0,8 mm Sieb getrieben werden. Nach 24 Stunden Trocknen bei Raumtemperatur in einem Abzug werden die Partikel zusammen mit dem Magnesiumstearat und Cayennepfeffer durch ein 1,0 mm Sieb getrieben und in einem Containermischer gemischt.

**Push-Schicht**

| | |
|---|---|
| Methylhydroxypropylcellulose 6 mPas | 13 mg |
| Natriumchlorid | 80 mg |
| Macrogol 7 000 000 | 166 mg |
| Magnesiumstearat | 1 mg |

Natriumchlorid, Macrogol und die Hälfte der Methylhydroxypropylcellulose werden in einem Wirbelschichtgranulator 3 Minuten trocken gemischt und anschließend durch Aufsprühen einer Lösung der zweiten Hälfte der Methylhydroxypropylcellulose in 75 mg unter Zufuhr von Warmluft granuliert und getrocknet. Das Granulat wird anschließend zusammen mit dem Magnesiumstearat durch ein 2,5 mm Sieb in einer Comil getrieben.

**Emetikaschicht**

| | |
|---|---|
| Emetin | 50 mg |
| Hydriertes Rizinusöl (Cutina HR) | 50 mg |

Emetin und Hydriertes Rizinusöl werden in einer Tablettenpresse mit einem 10 mm Vorpreßstempel zu Preßlingen von ca. 250 mg vorgepreßt. Anschließend werden die Vorpreßlinge mittels eines Brechers und eines Siebes von 1,0 mm zerkleinert.

### Herstellung der 3-Schichttabletten

Pro Tablette werden 100 mg des Granulates der Emetikaschicht, 260 mg der Push-Schicht und 450 mg der Wirkstoffschicht werden nacheinander in die Matrize einer geeigneten Tablettenpresse gefüllt und zu einer 3-Schichttablette verpreßt.

**Überzug auf Kern**

| | |
|---|---|
| Celluloseacetat mit 39,8 % Acetat | 38 mg |
| Macrogol 3350 | 2 mg |

Die Überzugsbestandteile werden in einem Aceton-Wasser Gemisch (95:5 Gewichtsteile) als 3,8% ige Lösung gelöst und auf die Kerne gesprüht. Durch den Überzug werden zwei Löcher von 0,75 mm gebohrt um die Wirkstoffschicht mit der äußeren Umgebung des Systems zu verbinden.

### Beispiel 26

### Orales osmotisches therapeutisches System

Es wird wie bei Beispiel 25 vorgegangen mit dem Unterschied, daß die Emetikaschicht folgende Zusammensetzung aufweist:

| | |
|---|---|
| Emetinhydrochlorid-Pentahydrat | 60 mg |
| Hydriertes Rizinusöl (Cutina HR) | 40 mg |

Emetinhydrochlorid-Pentahydrat und Hydriertes Rizinusöl werden in einer Tablettenpresse mit einem 10 mm Vorpreßstempel zu Preßlingen von ca. 250 mg vorgepreßt Anschließend werden die Vorpreßtinge mittels eines Brechers und eines Siebes von 1,0 mm zerkleinert.

Alle übrigen Herstellschritte erfolgen wie in Beispiel 25 dargestellt

### Beispiel 27

### Manteltabletten

**Kern**

| | |
|---|---|
| Naltrexonhydrochlorid | 50 mg |
| Sprühgetrocknete Lactose | 46 mg |
| Magnesiumstearat | 2 mg |
| Kolloidales Siliciumdioxid | 2 mg |

Alle Bestandteile werden gemischt und auf einer Tablettenpresse zu runden, bikonvexen Tabletten mit 6,5 mm Durchmesser verpreßt.

**Überzug auf Kern**

| | |
|---|---|
| Celluloseacetat mit 39,8 % Acetat | 9,5 mg |
| Macrogol 3350 | 0,5 mg |

Die Überzugsbestandteile werden in einem Aceton-Wasser Gemisch (95:5 Gewichtsteile) gelöst und auf die Kerne gesprüht.

**Mantel**

| | |
|---|---|
| Morphinsulfat Pentahydrat | 60 mg |
| 100 000 mPas (Metolose 90 SH 100 000, ShinEtsu) | 100 mg |
| Xanthan, NF | 40 mg |
| Mikrokristalline Cellulose (Avicel PH 102) | 165 mg |
| Lactose Monohydrat | 155 mg |
| Cayennepfeffer | 10 mg |
| Magnesiumstearat | 5 mg |
| Kolloidales Siliciumdioxid | 5 mg |

Alle Mantelbestandteile werden gemischt, in einer Tablettenpresse mit Werkzeug für 13 mm bikonvexe Tabletten wurden ca. 270 mg der Mischung in die Tablettenmatrize gefüllt, der mit Celluloseacetat überzogene Kern zentriert eingelegt, die restlichen 270 mg der Mantelmischung zugefüllt und der Mantel um den Kern gepreßt.

### Beispiel 28

### Multipartikuläre Form

**Emetika Pellets**

| | |
|---|---|
| Emetin | 50 mg |
| Lactose | 15 mg |
| Mikrokristalline Cellulose PH101 | 30 mg |
| Niedrigsubstituierte Hydroxypropylcellulose (LH31, Shin-Etsu) | 5 mg |

Alle Bestandteile werden in einem geeigneten Mischer ca. 5 min innig miteinander vermischt. Während des Mischens wird die Mischung mit einer solchen Menge Gereinigtem Wasser granuliert, daß sich eine feuchte granulierte Masse bildet. Das so erhaltene Granulat wird in einem Nica-Extruder durch eine Matrize mit Extrusionsöffnungen von 1 mm extrudiert, 5 min auf einem Spheronizer gerundet, in der Wirbelschicht bei 60°C getrocknet und mittels eines 1,5 mm und eines 0,5 mm Siebs klassiert.

**Überzug auf Emetika Pellets**

| | |
|---|---|
| Celluloseacetat mit 39,8 % Acetat | 9,5 mg |
| Macrogol 3350 | 0,5 mg |
| Titandioxid | 0,5 mg |

Mengenangaben pro 100 mg Emetikapellets

Celluloseacetat und Macrogol werden in einem Aceton-Wasser Gemisch (95:5 Gewichtsteile) 3,8% ige Lösung gelöst, Titandioxid wird in der Mischung dispergiert und die Kerne in einer Wirbelschichtanlage mit der Suspension besprüht bis die Masse der überzogenen Pellets 110% des Gewichts der eingesetzten nicht überzogenen Pellets beträgt.

**Analgetica Pellets**

| | |
|---|---|
| Nonpareils 0,5 mm (Saccharose-Maisstärke Starter Pellets, Fa. Werner) | 50 mg |
| Morphinsulfat Pentahydrat | 60 mg |
| Povidon K 30 | 30 mg |
| Capsaicin | 0,1 mg |
| Talkum | 9,9 mg |
| Xanthan | 30 mg |

Morphinsulfat und Povidon werden in Gereinigtem Wasser gelöst und die Hälfte des Talkums wird in der Lösung dispergiert. Capsaicin wird als 10%ige Lösung in Ethanol gelöst und die Lösung wird der Suspension zugegeben. Die Suspension wird in bei 55°C in einem Rotogranulator (Fa. Glatt) auf die rotierenden Nonpareils aufgesprüht, das Xanthan wird als Pulver im Gemisch mit der zweiten Hälfte des Talkums in die Masse der befeuchteten, rotierenden Pellets kontinuierlich zugeführt. Nach Abschluß der Trocknung werden die Pellets mittels eines 1,5 mm und eines 0,5 mm Siebs klassiert.

**Überzug auf Analgetica Pellets**

| | |
|---|---|
| Ethylcellulose Dispersion (Aquacoat ECD 30, FMC Corporation) | 12,0 mg |
| Glycerolmonostearat | 2,4 mg |
| Talkum | 2,4 mg |
| Titandioxid | 1,2 mg |

Mengenangaben pro 180 mg Analgetica Pellets, Ethylcellulosegewichtsangabe als Trockenmasse aus der 30%igen Dispersion des Handelsprodukts.

Ethylcellulose Dispersion wird 1:0,5 mit Gereinigtem Wasser gemischt und das Glycerolmonostearat wird unter mindestens zwei stündigem Rühren eingearbeitet. Talkum und Titandioxid werden in 0,5 Teilen Wasser (Berechnungsbasis aus der 1:0,5 Mischung der Ethylcellulose Dispersion) dispergiert und mit der Ethylcellulosedispersion gemischt. Die Analgetica Pellets werden in einer Wirbelschichtanlage mit der Dispersion besprüht bis die Masse der überzogenen Pellets 110% des Gewichts der eingesetzten nicht überzogenen Pellets beträgt.

### Kapseln

Pro Kapsel werden jeweils 110 mg überzogene Emetika Pellets und 198 mg überzogene Analgetica Pellets gemischt und in Hartgelatinekapseln der Größe 1 abgefüllt.

### Beispiel 29

### Multipartikuläre Form

**Emetika Pellets**

| | |
|---|---|
| Emetin | 50 mg |
| Lactose | 15 mg |
| Mikrokristalline Cellulose PH101 | 30 mg |
| Niedrigsubstituierte Hydroxypropylcellulose (LH31, Shin-Etsu) | 5 mg |

Alle Bestandteile werden in einem geeigneten Mischer ca. 5 min innig miteinander vermischt. Während des Mischens wird die Mischung mit einer solchen Menge Gereinigtem Wasser granuliert, daß sich eine feuchte granulierte Masse bildet. Das so erhaltene Granulat wird in einem Nica-Extruder durch eine Matrize mit Extrusionsöffnungen von 1 mm extrudiert, 5 min auf einem Spheronizer gerundet, in der Wirbelschicht bei 60°C getrocknet und mittels eines 1,5 mm und eines 0,5 mm Siebs klassiert.

**Überzug auf Emetika Pellets**

| | |
|---|---|
| Celluloseacetat mit 39,8 % Acetat | 9,5 mg |
| Macrogol 3350 | 0,5 mg |
| Titandioxid | 0,5 mg |

Mengenangaben pro 100 mg Emetikapellets

Celluloseacetat und Macrogol werden in einem Aceton-Wasser Gemisch (95:5 Gewichtsteile) 3,8% ige Lösung gelöst, Titandioxid wird in der Mischung dispergiert und die Kerne in einer Wirbelschichtanlage mit der Suspension besprüht bis die Masse der überzogenen Pellets 110% des Gewichts der eingesetzten nicht überzogenen Pellets beträgt.

**Antagonisten Pellets**

| | |
|---|---|
| Naloxonhydrochlorid-Dihydrat | 20 mg |
| Lactose | 7 mg |
| Mikrokristalline Cellulose PH101 | 20 mg |
| Niedrigsubstituierte Hydroxypropylcellulose (LH31, Shin-Etsu) | 3 mg |

Alle Bestandteile werden in einem geeigneten Mischer ca. 5 min innig miteinander vermischt. Während des Mischens wird die Mischung mit einer solchen Menge Gereinigtem Wasser granuliert, daß sich eine feuchte granulierte Masse bildet. Das so erhaltene Granulat wird in einem Nica-Extruder durch eine Matrize mit Extrusionsöffnungen von 1 mm extrudiert, 5 min auf einem Spheronizer gerundet, in der Wirbelschicht bei 60°C getrocknet und mittels eines 1,5 mm und eines 0,5 mm Siebs klassiert.

**Überzug auf Antagonisten Pellets**

| | |
|---|---|
| Celluloseacetat mit 39,8 % Acetat | 4,75 mg |
| Macrogol 3350 | 0,25 mg |
| Titandioxid | 0,25 mg |

Mengenangaben pro 50 mg Emetikapellets

Celluloseacetat und Macrogol werden in einem Aceton-Wasser Gemisch (95:5 Gewichtsteile) 3,8% ige Lösung gelöst, Titandioxid wird in der Mischung dispergiert und die Kerne in einer Wirbelschichtanlage mit der Suspension besprüht bis die Masse der überzogenen Pellets 110% des Gewichts der eingesetzten nicht überzogenen Pellets beträgt.

**Analgetica Pellets**

| | |
|---|---|
| Nonpareils 0,5 mm (Saccharose-Maisstärke Starter Pellets, Fa. Werner) | 50 mg |
| Morphinsulfat Pentahydrat | 60 mg |
| Povidon K 30 | 30 mg |
| Capsaicin | 0,1 mg |
| Talkum | 9,9 mg |
| Xanthan | 30 mg |

Morphinsulfat und Povidon werden in Gereinigtem Wasser gelöst und die Hälfte des Talkums wird in der Lösung dispergiert. Capsaicin wird als 10%ige Lösung in Ethanol gelöst und die Lösung wird der Suspension zugegeben. Die Suspension wird in bei 55°C in einem Rotogranulator (Fa. Glatt) auf die rotierenden Nonpareils aufgesprüht, das Xanthan wird als Pulver im Gemisch mit der zweiten Hälfte des Talkums in die Masse der befeuchteten, rotierenden Pellets kontinuierlich zugeführt. Nach Abschluß der Trocknung werden die Pellets mittels eines 1,5 mm und eines 0,5 mm Siebs klassiert.

**Überzug auf Analgetica Pellets**

| | |
|---|---|
| Ethylcellulose Dispersion (Aquacoat ECD 30, FMC Corporation) | 12,0 mg |
| Glycerolmonostearat | 2,4 mg |
| Talkum | 2,4 mg |
| Titandioxid | 1,2 mg |

Mengenangaben pro 180 mg Analgetica Pellets, Ethylcellulosegewichtsangabe als Trockenmasse aus der 30%igen Dispersion des Handelsprodukts.

Ethylcellulose Dispersion wird 1:0,5 mit Gereinigtem Wasser gemischt und das Glycerolmonostearat wird unter mindestens zwei stündigem Rühren eingearbeitet. Talkum und Titandioxid werden in 0,5 Teilen Wasser (Berechnungsbasis aus der 1:0,5 Mischung der Ethylcellulose Dispersion) dispergiert und mit der Ethylcellulosedispersion gemischt. Die Analgetica Pellets werden in einer Wirbelschichtanlage mit der Dispersion besprüht bis die Masse der überzogenen Pellets 110% des Gewichts der eingesetzten nicht überzogenen Pellets beträgt.

### Kapseln

Pro Kapsel werden jeweils 110 mg überzogene Emetika Pellets, 55 mg Antagonisten Pellets und 198 mg überzogene Analgetica Pellets mit Gelbildner gemischt und in Hartgelatinekapseln der Größe 0 abgefüllt.

## Patentansprüche

1. Gegen Mißbrauch gesicherte Darreichungsform, **dadurch gekennzeichnet, daß** sie neben einem oder mehreren Wirkstoffen mit Mißbrauchspotential zwei oder mehr der nachfolgenden Komponenten a)-d) aufweist:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wäßrigen Flüssigkeit aus der Darreichungsform gewonnenen Extrakt ein Gel bildet, welches beim Einbringen in eine weitere Menge einer wäßrigen Flüssigkeit visuell unterscheidbar bleibt,
(c) wenigstens einen Antagonisten für Wirkstoff bzw. die Wirkstoffe mit Mißbrauchspotential
(d) wenigstens ein Emetikum.

2. Darreichungsform gemäß Anspruch 1, **dadurch gekennzeichnet, daß** sie drei der Komponenten (a)-(d) aufweist, vorzugsweise (a), (b) und (c) oder (a), (b) und (d).

3. Darreichungsform gemäß Anspruch 1, **dadurch gekennzeichnet, daß** sie sämtliche Komponenten (a)-(d) aufweist.

4. Darreichungsform gemäß Anspruch 1 oder 2 enthaltend zwei oder drei der Komponenten (a), (b) und (d), **dadurch gekennzeichnet, daß** der Wirkstoff ausgewählt ist aus der Gruppe bestehend aus Opiaten, Opioiden, Tranquillantien, Stimulantien und weiteren Betäubungsmitteln.

5. Darreichungsform gemäß Anspruch 4, **dadurch gekennzeichnet, daß** der Wirkstoff ausgewählt ist aus der Gruppe bestehend aus N-{1-[2-(4-Ethyl-5-oxo-2-tetrazolin-1-yl)ethyl]-4-methoxymethyl-4-piperidyl}propionanilid (Alfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-α-Methylphenethylamin (Amfetamin), 2-(α-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3*H*)-on (Bromazepam), 2-Brom-4-(2-chlorphenyl)-9-methyl-6*H*-thieno[3,2-*f*][1,2,4]triazolo[4,3-a][1,4]diazepin (Brotizolam), 17-Cyclopropylmethyl-4,5α-epopxy-7α[(S)-1-hydroxy-1,2,2-trimethyl-propyl]-6-methoxy-6,14-*endo-*ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H-*1,4-benzodiazepin-3-yl)-dimethyl-carbamat (Camazepam), (1*S*,2*S*)-2-Amino-1-phenyl-1-propanol (Cathin / D-Norpseudoephedrin), 7-Chlor-*N*-methyl-5-phenyl-3*H*-1,4-benzodiazepin-2-ylamin-4-oxid (Chlordiazepoxid), 7-Clor-1-methyl-5-phenyl-1*H*-1,5-benzodiazepin-2,4(3*H*,5*H*)-dion (Clobazam), 5-(2-Chlorphenyl)-7-nitro-1*H*-1,4-benzodiazepin-2(3*H*)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H*-thieno[2,3-e][1,4]diazepin-2(3*H*)-on (Clotiazepam), 10-Chlor-11b-(2chlorphenyl)-2,3,7,11 1b-tetrahydrooxazolo[3,2-*d*][1,4]benzodiazepin-6(5H)-on (Cloxazolam), (-)-Methyl-[3β-benzoyloxy-2β(1αH,5α*H*)-tropancarboxylat] (Cocain), 4,5α-Epoxy-3-methoxy-17-methyl-7-morphinen-6α-ol (Codein), 5-(1 -cyclohexenyl)-5-ethylbarbitursäure (Cyclobarbital), Cyclorphan, Cyprenorphin, 7-Chlor-5-(2-chlorphenyl)-1*H*-1,4-benzodiazepin-2(3H)-on (Delorazepam), Desomorphin, Dextromoramid, (+)-(1-Benzyl-3-dimethylamino-2-methyl-1-phenylpropyl)propionat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5α-Epoxy-3-methoxy-17-methyl-6α-morphinanol (Dihydrocodein), 4,5□-Epoxy-17-methyl-3,6a-morphinandiol (Dihydromorphin), Dimenoxadol, Dimephetamol, Dimethylthiambuten, Dioxaphetylbutyrat, Dipipanon, (6a*R*,10a*R*)-6,6,9-Trimethyl-3-pentyl-6a,7,8,10a-tetrahydro-6*H-*benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H-*1,4 benzodiazepin-3-carboxylat] (Ethylloflazepat), 4,5□-Epoxy-3-ethoxy-17-methyl-7-morphinen-6□-ol (Ethylmorphin), Etonitazen, 4,5α-Epoxy-7α-(1-hydroxy-1-methylbutyl)-6-methoxy-17-methyl-6,14-endo-etheno-morphinan-3-ol (Etorphin), *N*-Ethyl-3-phenyl-8,9,10-trinorbornan-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1H-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1*H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3*S*,6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 6-(2-Chlorphenyl)-2-(4-methyl-1-piperazinylmethylen)-8-nitro-2*H*-imidazo[1,2-a][1,4] benzodiazepin-1(4*H*)-on (Loprazolam), 7-Chlor-5-(2-chlorphenyl)-3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H-*imidazo[2,1-a]isoindol-5-ol (Mazindol), 7-Chlor-2,3-dihydro-1-methyl-5-phenyl-1*H*-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-o-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-a][1,4]benzodiazepin (Midazolam), 2-(Benzhydrylsulfinyl)acetamid (Modafinil), 4,5a-Epoxy-17-methyl-7-morphinen-3,6a-diol (Morphin), Myrophin, (±)-*trans*-3-(1,1-Dimethylheptyl)-7,8,10,10α-tetrahydro-1-hydroxy-6,6-dimethyl-6*H* dibenzo [b, d]pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3H)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*)-on (Oxazepam), (*cis*-*trans*)*-*10-Chlor-2,3,7,11b-tetrahydro-2-methyl-11b-phenyloxazolo[3,2-*d*][1,4]benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pernolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-phenyl-1*H*-1,4-benzodiazepin-2(3*H*)-on (Prazepam), Profadol, Proheptazin, Promedol, Properidin, Propoxyphen, N-(1-Methyl-2-piperidinoethyl)-*N*-(2-pyridyl)propionamid, Methyl{3-[4-methoxycarbonyl-4-(*N*-phenylpropanamido)piperidino]propanoat} (Remifentanil), 5-*sec*-Butyl-5-ethylbarbitursäure (Secbutabarbital), 5-Allyl-5-(1-methylbutyl)-barbitursäure (Secobarbitat), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3*H*)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Tramadol, 8-Chlor-6-(2-chlorphenyl)-1-methyl-4*H*-[1,2,4]triazolo[4,3-a][1,4]benzodiazepin (Triazolam), 5-(1-Methylbutyl)-5-vinylbarbitursäure (Vinylbital), (1R*,2R*)-3-(3-Dimethylamino-1-ethyl-2-methyl-propyl)-phenol, (1 R, 2R, 4S)-2-[Dimethylamino)methyl-4-(p-fluorbenzyloxy)-1-(m-methoxyphenyl)cyclohexanol, jeweils ggf. in Form entsprechender stereoisomerer Verbindungen sowie entsprechender Derivate, insbesondere Ester oder Ether, und jeweils physiologisch verträglicher Verbindungen, insbesondere Salze und Solvate.

6. Darreichungsform gemäß einem der Ansprüche 1 bis 3 enthaltend die Komponente (c), **dadurch gekennzeichnet, daß** der Wirkstoff ausgewählt ist aus der Gruppe bestehend aus Opiaten, Opioiden, Stimulantien und weiteren Betäubungsmitteln.

7. Darreichungsform gemäß Anspruch 6, **dadurch gekennzeichnet, daß** der Wirkstoff ausgwählt worden ist aus der Gruppe bestehend aus bestehend aus N-{1-[2-(4-Ethyl-5-oxo-2-tetrazolin-1-yl)ethyl]-4-methoxymethyl-4-piperidyl}propionanilid (Alfentanil), Allylprodin, Alphaprodin, 2-Diethylaminopropiophenon (Amfepramon), (±)-α-Methylphenethylamin (Amfetamin), 2-(α-Methylphenethylamino)-2-phenylacetonitril (Amfetaminil), Anileridin, Apocodein, Benzylmorphin, Bezitramid, 17-Cyclopropylmethyl-4,5α-epopxy-7α[(S)-1-hydroxy-1,2,2-trimethyl-propyl]-6-methoxy-6,14-*endo-*ethanomorphinan-3-ol (Buprenorphin), Butorphanol, (1*S*,2*S*)-2-Amino-1-phenyl-1-propanol (Cathin / D-Norpseudoephedrin), Clonitazen, (-)-Methyl-[3β-benzoyloxy-2β(1αH,5α*H*)-tropancarboxylat] (Cocain), 4,5α-Epoxy-3-methoxy-17-methyl-7-morphinen-6α-ol (Codein), Cyclorphan, Cyprenorphin, Desomorphin, Dextromoramid, (+)-(1-Benzyl-3-dimethylamino-2-methyl-1-phenylpropyl)propionat (Dextropropoxyphen), Dezocin, Diampromid, Diamorphon, 4,5α-Epoxy-3-methoxy-17-methyl-6α-morphinanol (Dihydrocodein), 4,5□-Epoxy-17-methyl-3,6a-morphinandiol (Dihydromorphin), Dimenoxadol, Dimephetamol, Dimethylthiambuten, Dioxaphetylbutyrat, Dipipanon, (6a*R*,10a*R*)-6,6,9-Trimethyl-3-pentyl-6a,7,8,10a-tetrahydro-6*H-*benzo[c]chromen-1-ol (Dronabinol), Eptazocin, Ethoheptazin, Ethylmethylthiambuten, 4,5□-Epoxy-3-ethoxy-17-methyl-7-morphinen-6□-ol (Ethylmorphin), Etonitazen, 4,5α-Epoxy-7a-(1-hydroxy-1-methylbutyl)-6-methoxy-17-methyl-6,14-*endo*-etheno-morphinan-3-ol (Etorphin), *N*-Ethyl-3-phenyl-8,9,10-trinorbornan-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 1-[4-(3-Hydroxyphenyl)-1-methyl-4-piperidyl]-1-propanon (Ketobemidon), (3S,6S)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-17-Methyl-3-morphinanol (Levorphanol), Levophenacylmorphan, Lofentanil, 5-(4-Chlorphenyl)-2,5-dihydro-3*H*-imidazo[2,1-a]isoindol-5-ol (Mazindol), *N*-(3-Chlorpropyl)-α-methylphenethylamin (Mefenorex), Meperidin, Meptazinol, Metazocin, Methylmorphin, N,α-Dimethylphenethylamin (Metamfetamin), (±)-6-Dimethylamino-4,4-diphenyl-3-heptanon (Methadon), Methyl-[2-phenyl-2-(2-piperidyl)acetat] (Methylphenidat), 3,3-Diethyl-5-methyl-2,4-piperidindion (Methyprylon), 2-(Benzhydrylsulfinyl)acetamid (Modafinil), 4,5α-Epoxy-17-methyl-7-morphinen-3,6α-diol (Morphin), Myrophin, (±)-*trans*-3-(1,1-Dimethylheptyl)-7,8,10,10α-tetrahydro-1-hydroxy-6,6-dimethyl-6*H*-dibenzo [*b*, *d*]pyran-9(6αH)-on (Nabilon), Nalbuphen, Narcein, Nicomorphin, Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pernolin), 1,2,3,4,5,6-Hexahydro-6,11-dimethyl-3-(3-methyl-2-butenyl)-2,6-methano-3-benzazocin-8-ol (Pentazocin), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), α,α-Dimethylphenethylamin (Phentermin), α-(2-Piperidyl)benzhydrylalkohol (Pipradrol), 1'-(3-Cyan-3,3-diphenylpropyl)[1,4'-bipiperidin]-4'-carboxamid (Piritramid), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), *N*-{4-Methoxymethyl-1-[2-(2-thienyl)ethyl]-4-piperidyl}propionanilid (Sufentanil), Ethyl-(2-dimethylamino-1-phenyl-3-cyclohexen-1-carboxylat) (Tilidin (cis und trans)), Tramadol, (1R*,2R*)-3-(3-Dimethylamino-1-ethyl-2-methyl-propyl)-phenol, (1 R, 2R, 4S)-2-[Dimethylamino)methyl-4-(p-fluorbenzyloxy)-1-(m-methoxyphenyl)cyclohexanol, jeweils ggf. in Form entsprechender stereoisomerer Verbindungen sowie entsprechender Derivate, insbesondere Ester oder Ether, und jeweils physiologisch verträglicher Verbindungen, insbesondere Salze und Solvate.

8. Darreichungsform gemäß einem der Ansprüche 4 bis 7, **dadurch gekennzeichnet, daß** sie ein oder mehrere Stimulantien enthält, ausgewählt aus der Gruppe bestehend aus Amphetamin, Norpseudoephedrin, Methylphenidat und jeweils ggf. deren entsprechenden physiologisch verträglichen Verbindungen, insbesondere deren Basen, Salzen und Solvaten.

9. Darreichungsform gemäß einem der Ansprüche 1 bis 8, **dadurch gekennzeichnet, daß** der reizende Stoff der Komponente (a) ein Brennen, einen Juckreiz, einen Niesreiz, eine vermehrte Sekretbildung oder eine Kombination mindestens zweier dieser Reize verursacht.

10. Darreichungsform gemäß einem der Ansprüche 1 bis 9, **dadurch gekennzeichnet, daß** der reizende Stoff der Komponente (a) auf einem oder mehreren Inhaltsstoffen wenigstens einer Scharfstoffdroge basiert.

11. Darreichungsform gemäß Anspruch 10, dadurch gekennzelchnet, daß die Scharrstofrdroge ausgewählt ist aus der Gruppe bestehend aus Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhdoma, besonders bevorzugt aus der Gruppe bestehend aus Capsici Fructus (Paprika), Capsici Fructus acer (Cayennepfeffer) und Piperis nigri Fructus (Pfeffer).

12. Darreichungsform gemäß Anspruch 10 oder 11, **dadurch gekennzeichnet, daß** der Inhaftsstoff eine o-Methoxy(Methyl)-phenol-Verbindung, eine Säureamid-Verbindung, ein Senföl oder eine Sulfidverbindung ist oder sich von einer solchen Verbindung ableitet

13. Darreichungsform gemäß einem der Ansprüche 10 bis 12, **dadurch gekennzeichnet, daß** der Inhaltsstoff ausgewählt worden ist aus der Gruppe bestehend aus Myristicin, Elemicin, Isoeugenol, β Asaron, Safroi, Gingerolen, Xanthorrhizol, Gapsaicinoiden, vorzugsweise Capsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmerlaptosenföl oder Methylsulfonylsenföl, und von diesen Inhaltsstoffen abgeleiteten Verbindungen.

14. Darreichungsform gemäß einem der Ansprüche 1 bis 13, **dadurch gekennzeichnet, daß** die Komponente (b) ein oder mehrere viskositätserhöhende Mittel aufweist, ausgewählt aus der Gruppe bestehend aus mikrokristalliner Cellulose mit 11 Gel.-% Carboxymethylcellulose-Natrium (Avicel^{®} RC 591), Carboxymethylcellulose-Natrium (Blanose^{®}, CMC-Na C300P^{®}, Frimulsion BLC-5^{®}. Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Citrus-Pectin (Cesapectin^{®} HM Medium Rapid Set), Wachsmaisstärke (C*Gel 04201^{®}), Natriumalginat (Frimulsion ALG (E401)^{®}), Guarkemmehl (Frimulsion BM^{®}, Polygum 26/1-75^{®}), Iota-Carrageen (Frimulsion D021^{®}), Karaya Gummi, Gellangummi (Kelcogel F^{®}, Kelcogel LT100^{®}), Galaktomannan (Meyprogat 150^{®}), Tarakemmehl (Polygum 43/1^{®}), Propylenglykoalginat (Protanal-Ester SD-LB^{®}), Apfelpektin, Pektin aus Zitronenschale, Natrium-Hyaluronat, Tragant, Taragummi (Vidogum SP 200^{®}), fermentiertes Polysaccharid- Welan Gum (K1A96) und Xanthan-Gummi (Xantural 180^{®}).

15. Darreichungsform gemäß einem der Ansprüche 1 bis 14, **dadurch gekennzeichnet, daß** sie die viskositätserhöhenden Mittel in einer Menge von ≥ 5 mg pro Darreichungsform, d.h. pro Dosiereinheit aufweist.

16. Darreichungsform gemäß einem der Ansprüche 7 bis 15, **dadurch gekennzeichnet, daß** als Antagonist der Komponente (c) ein Opiat- oder Opioid-Antagonist ausgewählt aus der Gruppe bestehend aus Naloxon, Naltrexon, Nalmefen, Nalid. Nalmexon, Nalorphin, Naluphin und jeweils entsprechender physiologisch verträglicher Verbindungen, insbesondere Basen, Salze und Solvate, zum Einsatz kommt.

17. Darreichungsform gemäß einem der Ansprüche 4 bis 16, **dadurch gekennzeichnet, daß** als Antagonist der Komponente (c) für ein Stimulanz ein Neuroleptikum, vorzugsweise ausgewählt aus der Gruppe bestehend aus Haloperidol, Promethacin, Fluphenazin, Perphenazin, Levomepromazin, Thioridazin, Perazin, Chlorpromazin, Chlorprothixin, Zuclopentixol, Flupentixol, Prothipendyl, Zotepin, Benperidol, Pipamperon, Melperon und Bromperidol zum Einsatz kommt.

18. Darreichungsform gemäß einem der Ansprüche 1 bis 17, **dadurch gekennzeichnet, daß** das Emetikum der Komponente (d) auf einem oder mehreren Inhaltsstoffen von Radix Ipecacuanhae (Brechwurzel), vorzugsweise auf dem Inhaltsstoff Emetin basiert, und/oder Apomorphin ist.

19. Darreichungsform gemäß einem der Ansprüche 1 bis 18, **dadurch gekennzeichnet, daß** der Wirkstoff bzw. die Wirkstoffe von der Komponente (c) und/oder (d) räumlich getrennt ist, wobei der Wirkstoff bzw. die Wirkstoffe bevorzugt in wenigstens einer Untereinheit (A) und die Komponenten (c) und/oder (d) in wenigstens einer Untereinheit (B) vorliegen und die Komponenten (c) und/oder (d) aus der Untereinheit (B) bei bestimmungsgemäßer Applikation der Darreichungsform im Körper nicht ihre Wirkung entfalten.

20. Darreichungsform gemäß Anspruch 19, **dadurch gekennzeichnet**, **dadurch gekennzeichnet, daß** beide der Untereinheiten (A) und (B) in multipartikulärer Form, vorzugsweise in Form von Mikrotabletten, Mikrokapseln, Mikropellets, Granulaten, Sphäroiden, Perlen oder Pellets, ggf. zu Tabletten verpreßt, in Kapseln abgefüllt oder in einer Flüssigkeit oder einem Gel suspendiert, vorliegen, wobei sowohl für die Untereinheit (A) als auch (B) dieselbe Form, d.h. Gestaltung gewählt wird.

21. Darreichungsform gemäß Anspruch 20, **dadurch gekennzeichnet, daß** die jeweiligen weitgehend identisch gestalteten multipartikulären Formen der Untereinheit (A) bzw (B) auch visuell nicht unterscheidbar sind.

22. Darreichungsform gemäß Anspruch 19, **dadurch gekennzeichnet, daß** die Untereinheiten (A) und (B) jeweils schichtförmig zueinander angeordnet sind.

23. Darreichungsform gemäß Anspruch 22, **dadurch gekennzeichnet, daß** die schichtförmigen Untereinheiten (A) und (B) vertikal oder horizontal zueinander angeordnet sind.

24. Darreichungsform gemäß Anspruch 22, **dadurch gekennzeichnet, daß** die Untereinheit (B) einen Kern bildet, der von der Untereinheit (A) vollständig umhüllt wird.

25. Darreichungsform gemäß Anspruch 22, **dadurch gekennzeichnet, daß** die Untereinheit (A) einen Kern bildet, der von der Untereinheit (B) umhüllt wird, wobei diese Umhüllung wenigstens einen Kanal aufweist, der von dem Kern an die Oberfläche der Darreichungsform führt.

26. Darreichungsform gemäß einem der Ansprüche 22 bis 25, **dadurch gekennzeichnet, daß** zwischen den Schichten der Untereinheiten (A) und (B) wenigstens eine, ggf. quellbare Trennschicht (C) angeordnet ist.

27. Darreichungsform gemäß Anspruch 22 oder 26, **dadurch gekennzeichnet, daß** sie in Form einer Tablette vorliegt.

28. Darreichungsform gemäß einem der Ansprüche 22, 23, 26 oder 27, **dadurch gekennzeichnet**, die freie Oberfläche der Untereinheit (B) vollständig und ggf. zumindest ein Teil der freien Oberfläche der Untereinheit (A) und ggf. zumindest ein Teil der freien Oberfläche der Trennschicht (C) mit wenigstens einer die Freisetzung der Komponente (c) und/oder (d) verhindernden Barriereschicht (D) überzogen ist.

29. Darreichungsform gemäß einem der Ansprüche 22, 23, 26 oder 27, **dadurch gekennzeichnet, daß** sie zwischen den Untereinheiten (A) und (B) eine Push-Schicht (P) aufweist und sämtliche freie Oberflächen des Schichtaufbaus aus den Untereinheiten (A) und (B), der Push-Schicht (P) und und ggf. der Trennschicht (C) mit einem semipermeablen Überzug (E) ausgerüstet sind, der für das Freisetzungsmedium durchlässig, für den Wirkstoff und für die Komponente (c) und/oder (d) im wesentlichen undurchlässig ist, und wobei dieser Überzug (E) im Bereich der Untereinheit (A) wenigstens eine Öffnung zur Freisetzung des Wirkstoffes aufweist.

30. Darreichungsform gemäß Anspruch 19, **dadurch gekennzeichnet, daß** die Untereinheit (A) die Form einer Tablette hat, deren Steg und ggf. eine der beiden Grundflächen mit wenigstens einer das Emetikum enthaltenden Barrierschicht (D) bedeckt ist.

31. Darreichungsform gemäß einem der Ansprüche 1 bis 30, **dadurch gekennzeichnet, daß** sie wenigstens einen Wirkstoff zumindest teilweise in retardierter Form aufweist.

32. Darreichungsform gemäß einem der Ansprüche 1 bis 31 zur oralen Verabreichung.

33. Darreichungform gemäß Anspruch 32, **dadurch gekennzeichnet, daß** sie wenigstens einen magensaftresistenten Überzug aufweist.

34. Darreichungsform gemäß einem der Ansprüche 19 bis 33, **dadurch gekennzeichnet, daß** sie in wenigstens einer Untereinheit A und/oder wenigstens einer Untereinheit B die Komponente (a) und/oder (b) enthalten.

## Claims

1. Dosage form safeguarded against abuse, **characterised in that** in addition to one or more active ingredients with abuse potential it comprises two or more of the following components (a) - (d):
(a) at least one substance which irritates the nasal passages and/or pharynx;
(b) at least one viscosity-increasing agent, which, in an extract obtained from the dosage form with the assistance of a necessary minimum quantity of an aqueous liquid, forms a gel that remains visually distinguishable when introduced into a further quantity of an aqueous liquid,
(c) at least one antagonist for the active ingredient or ingredients with potential for abuse,
(d) at least one emetic.

2. Dosage form according to claim 1, **characterised in that** it has three of the components (a) - (d), preferably (a), (b) and (c) or (a), (b) and (d).

3. Dosage form according to claim 1, **characterised in that** it has all of the components (a) - (d).

4. Dosage form according to claim 1 or 2 containing two or three of the components (a), (b) and (d), **characterised in that** the active ingredient is selected from the group consisting of opiates, opioids, tranquillisers, stimulants and further narcotics.

5. Dosage form according to claim 4, **characterised in that** the active ingredient is selected from the group consisting of N-{1-[2-(4-ethyl-5-oxo-2-tetrazolin-1-yl)ethyl]-4-methoxymethyl-4-piperidyl}propionanilide (alfentanil), 5,5-diallylbarbituric acid (allobarbital), allylprodine, alphaprodine, 8-chloro-1-methyl-6-phenyl-4H-[1,2,4]triazolo[4,3-a][1,4]-benzodiazepine (alprazolam), 2-diethylamino-propiophenone (amfepramone), (±)-a-methylphenethylamine (amphetamine), 2-(α-methylphenethylamino)-2-phenylacetonitrile (amphetaminil), 5-ethyl-5-isopentyl-barbituric acid (amobarbital), anileridine, apocodeine, 5,5-diethylbarbituric acid (barbital), benzylmorphine, bezitramide, 7-bromo-5-(2-pyridyl)-1H-1,4-benzodiazepine-2(3H)-one (bromazepam), 2-bromo-4-(2-chlorophenyl)-9-methyl-6H-thieno[3,2-f][1,2,4]triazolo[4,3-a][1,4]diazepine (brotizolam), 17-cyclopropylmethyl-4,5α-epoxy-7α[(S)-1-hydroxy-1,2,2-trimethyl-propyl]-6-methoxy-6,14-endo-ethanomorphinan-3-ol (buprenorphine), 5-butyl-5-ethylbarbituric acid (butobarbital), butorphanol, (7-chloro-1,3-dihydro-1-methyl-2-oxo-5-phenyl-2H-1,4-benzodiazepin-3-yl) dimethylcarbamate (camazepam), (1S,2S)-2-amino-1-phenyl-1-propanol (cathine/D-norpseudoephedrine), 7-chloro-N-methyl-5-phenyl-3H-1,4-benzodiazepin-2-ylamine 4-oxide (chlordiazepoxide), 7-chloro-1-methyl-5-phenyl-1H-1,5-benzodiazepine-2,4(3H,5H)-dione (clobazam), 5-(2-chlorophenyl)-7-nitro- H-1,4-benzodiazepin-2(3H)-one (clonazepam), clonitazene, 7-chloro-2,3-dihydro-2-oxo-5-phenyl-1H-1,4-benzodiazepine-3-carboxylic acid (clorazepate), 5-(2-chlorophenyl)-7-ethyl-1-methyl-1H-thieno[2,3-e][1,4]diazepin-2(3H)-one(clotiazepam), 10-chloro-11b-(2-chlorophenyl)-2,3,7,11b-tetrahydrooxazolo[3,2-d][1,4]benzodiazepin-6(5H)-one (cloxazolam), (-)-methyl-[3β-benzoyloxy-2β(1αH,5αH)-tropancarboxylate] (cocaine), 4,5α-epoxy-3-methoxy-17-methyl-7-morphinan-6α-ol (codeine), 5-(1-cyclohexenyl)-5-ethylbarbituric acid (cyclobarbital), cyclorphan, cyprenorphine, 7-chloro-5-(2-chlorophenyl)-1H-1,4-benzodiazepin-2(3H)-one (delorazepam), desomorphine, dextromoramide, (+)-(1-benzyl-3-dimethylamino-2-methyl-1-phenylpropyl)propionate (dextropropoxyphen), dezocine, diampromide, diamorphone, 7-chloro-1-methyl-5-phenyl-1H-1,4-benzodiazepin-2(3H)-one (diazepam), 4,5α-epoxy-3-methoxy-17-methyl-6α-morphinanol (dihydrocodeine), 4,5α-epoxy-17-methyl-3,6a-morphinandiol (dihydromorphine), dimenoxadol, dimepheptanol, dimethylthiambutene, dioxaphetyl butyrate, dipipanone, (6aR,10aR)-6,6,9-trimethyl-3-pentyl-6a,7,8,10a-tetrahydro-6H-benzo[c]chromen-1-ol (dronabinol), eptazocine, 8-chloro-6-phenyl-4H-[1,2,4]triazolo[4,3-a][1,4]benzodiazepine (estazolam), ethoheptazine, ethylmethylthiambutene, ethyl [7-chloro-5-(2-fluorophenyl)-2,3-dihydro-2-oxo-1H-1,4-benzodiazepine-3-carboxylate] (ethyl loflazepate), 4,5α-epoxy-3-ethoxy-17-methyl-7-morphinen-6α-ol (ethylmorphine), etonitazene, 4,5α-epoxy-7α-(1-hydroxy-1-methylbutyl)-6-methoxy-17-methyl-6,14-endo-etheno-morphinan-3-ol (etorphine), N-ethyl-3-phenyl-8,9,10-trinorbornan-2-ylamine (fencamfamine), 7-[2-(α-methylphenethylamino)ethyl]-theophylline) (fenethylline), 3-(α-methylphenethylamino)propionitrile (fenproporex), N-(1-phenethyl-4-piperidyl)-propionanilide (fentanyl), 7-chloro-5-(2-fluorophenyl)-1-methyl-1H-1,4-benzodiazepin-2(3H)-one (fludiazepam), 5-(2-fluorophenyl)-1-methyl-7-nitro-1H-1,4-benzodiazepin-2(3H)-one (flunitrazepam), 7-chloro-1-(2-diethylaminoethyl)-5-(2-fluorophenyl)-1H-1,4-benzodiazepin-2(3H)-one (flurazepam), 7-chloro-5-phenyl-1-(2,2,2-trifluoroethyl)-1H-1,4-benzodiazepin-2(3H)-one (halazepam), 10-bromo-11b-(2-fluorophenyl)-2,3,7,11b-tetrahydro[1,3]oxazolyl[3,2-d][1,4]benzodiazepin-6(5H)-one (haloxazolam), heroin, 4,5α-epoxy-3-methoxy-17-methyl-6-morphinanone (hydrocodone), 4,5α-epoxy-3-hydroxy-17-methyl-6-morphinanone (hydromorphone), hydroxypethidine, isomethadone, hydroxymethyl morphinane, 11-chloro-8,12b-dihydro-2,8-dimethyl-12b-phenyl-4H-[1,3]oxazino[3,2-d][1,4]benzodiazepine-4,7(6H)-dione (ketazolam),1-[4-(3-hydroxyphenyl)-1-methyl-4-piperidyl]-1-propanone (ketobemidone), (3S,6S)-6-dimethylamino-4,4-diphenylheptan-3-yl acetate (levacetylmethadol (LAAM)), (-)-6-dimethylamino-4,4-diphenyl-3-heptanone (levomethadone), (-)-17-methyl-3-morphinanol (levorphanol), levophenacyl-morphane, lofentanil, 6-(2-chlorophenyl)-2-(4-methyl-1-piperazinylmethylene)-8-nitro-2H-imidazo[1,2-a][1,4]-benzodiazepin-1(4H)-one (loprazolam), 7-chloro-5-(2-chlorophenyl)-3-hydroxy-1H-1,4-benzodiazepin-2(3*H*)-one (lorazepam), 7-chloro-5-(2-chlorophenyl)-3-hydroxy-1-methyl-1H-1,4-benzodiazepin-2(3H)-one (lormetazepam), 5-(4-chlorophenyl)-2,5-dihydro-3H-imidazo[2,1-a]isoindol-5-ol (mazindol), 7-chloro-2,3-dihydro-1-methyl-5-phenyl-1H-1,4-benzodiazepine (medazepam), N-(3-chloropropyl)-α-methylphenethylamine (mefenorex), meperidine, 2-methyl-2-propyltrimethylene dicarbamate (meprobamate), meptazinol, metazocine, methylmorphine, N,α-dimethylphenethylamine (methamphetamine), (±)-6-dimethylamino-4,4-diphenyl-3-heptanone (methadone), 2-methyl-3-o-tolyl-4(3H)-quinazolinone (methaqualone), methyl [2-phenyl-2-(2-piperidyl)acetate] (methylphenidate), 5-ethyl-1-methyl-5-phenylbarbituric acid (methylphenobarbital), 3,3-diethyl-5-methyl-2,4-piperidinedione (methyprylon), metopon, 8-chloro-6-(2-fluorophenyl)-1-methyl-4H-imidazo[1,5-a][1,4]benzodiazepine (midazolam), 2-(benzhydrylsulphinyl)acetamide (modafinil), 4,5α-epoxy-17-methyl-7-morphinan-3,6α-diol (morphine), myrophine, (±)-trans-3-(1,1-dimethylheptyl)-7,8,10,10α-tetrahydro-1-hydroxy-6,6-dimethyl-6H-dibenzo[-b,d]pyran-9(6αH)-one (nabilone), nalbuphine, nalorphine, narceine, nicomorphine, 1-methyl-7-nitro-5-phenyl-1H-1,4-benzodiazepin-2(3H)-one (nimetazepam), 7-nitro-5-phenyl-1H-1,4-benzodiazepin-2(3H)-one (nitrazepam), 7-chloro-5-phenyl-1H-1,4-benzodiazepin-2(3H)-one (nordazepam), norlevorphanol, 6-dimethylamino-4,4-diphenyl-3-hexanone (normethadone), normorphine, norpipanone, latex from plants belonging to the species *Papaver somniferum* (opium), 7-chloro-3-hydroxy-5-phenyl-1H-1,4-benzodiazepin-2(3H)-one (oxazepam), (cis-trans)-10-chloro-2,3,7,11b-tetrahydro-2-methyl-11b-phenyloxazolo[3,2-d][1,4]benzodiazepin-6-(5H)-one(oxazolam),4,5α-epoxy-14-hydroxy-3-methoxy-7-methyl-6-morphinanone (oxycodone), oxymorphone, plants and plant parts belonging to the species *Papaver somniferum* (including the subspecies setigerum), papaveretum, 2-imino-5-phenyl-4-oxazolidinone (pemoline),1,2,3,4,5,6-hexahydro-6,11-dimethyl-3-(3-methyl-2-butenyl)-2,6-methano-3-benzazocin-8-ol(pentazocine), 5-ethyl-5-(1-methylbutyl)-barbituric acid (pentobarbital), ethyl (1-methyl-4-phenyl-4-piperidine carboxylate) (pethidine), phenadoxone, phenomorphan, phenazocine, phenoperidine, piminodine, pholcodine, 3-methyl-2-phenylmorpholine (phenmetrazine), 5-ethyl-5-phenylbarbituric acid (phenobarbital), α,α-dimethylphenethylamine (phentermine), 7-chloro-5-phenyl-1-(2-propynyl)-1H-1,4-benzodiazepin-2(3H)-one (pinazepam), α-(2-piperidyl)benzhydryl alcohol (pipradrol), 1'-(3-cyano-3,3-diphenylpropyl)[1,4'-bipiperidine]-4'-carboxamide (piritramide), 7-chloro-1-(cyclopropylmethyl)-5-phenyl-1H-1,4-benzodiazepin-2(3H)-one (prazepam), profadol, proheptazine, promedol, properidine, propoxyphene, N-(1-methyl-2-piperidinoethyl)-N-(2-pyridyl)-propionamide, methyl {3-[4-methoxycarbonyl-4-(N-phenylpropanamido)piperidino]-propanoate} (remifentanil), 5-sec-butyl-5-ethylbarbituric acid (secbutabarbital), 5-allyl-5-(1-methylbutyl)-barbituric acid (secobarbital), N-{4-methoxymethyl-l-[2-(2-thienyl)ethyl]-4-piperidyl}propionanilide (sufentanil), 7-chloro-2-hydroxy-methyl-5-phenyl-1H-1,4-benzodiazepin-2(3H)-one (temazepam), 7-chloro-5-(1-cyclohexenyl)-1-methyl-1H-1,4-benzodiazepin-2(3H)-one (tetrazepam), ethyl (2-dimethylamino-1-phenyl-3-cyclohexene-1-carboxylate) (tilidine (cis and trans)), tramadol, 8-chloro-6-(2-chlorophenyl)-1-methyl-4H-[1,2,4]triazolo[4,3-a][1,4]benzodiazepine (triazolam), 5-(1-methylbutyl)-5-vinylbarbituric acid (vinylbital), (1R*,2R*)-3-(3-dimethylamino-1-ethyl-2-methyl-propyl)-phenol, (1R,2R,4S)-2-(dimethylamino)methyl-4-(p-fluorobenzyloxy)-1-(m-methoxyphenyl)cyclohexanol, possibly in the form of respective corresponding stereoisomeric compounds and also corresponding derivatives, in particular esters or ethers, and respective physiologically acceptable compounds, in particular salts and solvates.

6. Dosage form according to one of claims 1 to 3 containing component (c), **characterised in that** the active ingredient is selected from the group consisting of opiates, opioids, stimulants and further narcotics.

7. Dosage form according to claim 6, **characterised in that** the active ingredient is selected from the group consisting of N-{1-[2-(4-ethyl-5-oxo-2-tetrazolin-1-yl)ethyl]-4-methoxymethyl-4-piperidyl}propionanilide (alfentanil), allylprodine, alphaprodine, 2-diethylamino-propiophenone (amfepramone), (±)-a-methylphenethylamine (amphetamine), 2-(α-methylphenethylamino)-2-phenylacetonitrile (amphetaminil), anileridine, apocodeine, benzylmorphine, bezitramide, 17-cyclopropylmethyl-4,5α-epoxy-7α[(S)-1-hydroxy-1,2,2-trimethyl-propyl]-6-methoxy-6,14-endo-ethanomorphinan-3-ol (buprenorphine), butorphanol, (1S,2S)-2-amino-1-phenyl-1-propanol (cathine/D-norpseudoephedrine), clonitazene, (-)-methyl-[3β-benzoyloxy-2β(1αH,5αH)-tropancarboxylate] (cocaine), 4,5α-epoxy-3-methoxy-17-methyl-7-morphinan-6α-ol (codeine), cyclorphan, cyprenorphine, desomorphine, dextromoramide, (+)-(1-benzyl-3-dimethylamino-2-methyl-1-phenylpropyl)-propionate (dextropropoxyphen), dezocine, diampromide, diamorphone, 4,5α-epoxy-3-methoxy-17-methyl-6α-morphinanol (dihydrocodeine), 4,5α-epoxy-17-methyl-3,6a-morphinandiol (dihydromorphine), dimenoxadol, dimepheptanol, dimethylthiambutene, dioxaphetyl butyrate, dipipanone, (6aR, 10aR)-6,6,9-trimethyl-3-pentyl-6a,7,8,10a-tetrahydro-6H-benzo[c]chromen-1-ol (dronabinol), eptazocine, ethoheptazine, ethylmethylthiambutene, 4,5α-epoxy-3-ethoxy-17-methyl-7-morphinen-6a-ol (ethylmorphine), etonitazene, 4,5α-epoxy-7α-(1-hydroxy-1-methylbutyl)-6-methoxy-17-methyl-6,14-endo-etheno-morphinan-3-ol (etorphine), N-ethyl-3-phenyl-8,9,10-trinorbonan-2-ylamine (fencamfamine), 7-[2-(α-methyl-phenethylamino)ethyl]-theophylline) (fenethylline), 3-(α-methyl-phenethylamino)propionitrile (fenproporex), N-(1-phenethyl-4-piperidyl)-propionanilide (fentanyl), heroin, 4,5α-epoxy-3-methoxy-17-methyl-6-morphinanone (hydrocodone), 4,5α-epoxy-3-hydroxy-17-methyl-6-morphinanone (hydromorphone), hydroxypethidine, isomethadone, hydroxymethyl morphinane, 1-[4-(3-hydroxyphenyl)-1-methyl-4-piperidyl]-1-propanone (ketobemidone), (3S,6S)-6-dimethylamino-4,4-diphenylheptan-3-yl acetate (levacetylmethadol (LAAM)), (-)-6-dimethylamino-4,4-diphenyl-3-heptanone (levomethadone), (-)-17-methyl-3-morphinanol (levorphanol), levophenacyl-morphane, lofentanil, 5-(4-chlorophenyl)-2,5-dihydro-3H-imidazo[2,1-a]isoindol-5-ol (mazindol), N-(3-chloropropyl)-α-methylphenethylamine (mefenorex), meperidine, meptazinol, metazocine, methylmorphine, N,α-dimethylphenethylamine (methamphetamine), (±)-6-dimethylamino-4,4-diphenyl-3-heptanone (methadone), methyl [2-phenyl-2-(2-piperidyl)acetate] (methylphenidate), 3,3-diethyl-5-methyl-2,4-piperidinedione (methyprylon), 2-(benzhydrylsulphinyl)acetamide (modafinil), 4,5α-epoxy-17-methyl-7-morphinan-3,6α-diol (morphine), myrophine, (±)-trans-3-(1,1-dimethylheptyl)-7, 8,10,1 0α-tetrahydro-1-hydroxy-6,6-dimethyl-6H-dibenzo[-b,d]pyran-9(6αH)-one (nabilone), nalbuphine, narceine, nicomorphine, norlevorphanol, 6-dimethylamino-4,4-diphenyl-3-hexanone (normethadone), normorphine, norpipanone, latex from plants belonging to the species *Papaver somniferum* (opium), 4,5α-epoxy-14-hydroxy-3-methoxy-17-methyl-6-morphinanone (oxycodone), oxymorphone, plants and plant parts belonging to the species *Papaver somniferum* (including the subspecies setigerum), papaveretum, 2-imino-5-phenyl-4-oxazolidinone (pemoline), 1,2,3,4,5,6-hexahydro-6,11-dimethyl-3-(3-methyl-2-butenyl)-2,6-methano-3-benzazocin-8-ol (pentazocine), ethyl (1-methyl-4-phenyl-4-piperidine carboxylate) (pethidine), phenadoxone, phenomorphan, phenazocine, phenoperidine, piminodine, pholcodine, 3-methyl-2-phenylmorpholine (phenmetrazine), α,α-dimethylphenethylamine (phentermine), α-(2-piperidyl)-benzhydryl alcohol (pipradrol), 1'-(3-cyano-3,3-diphenylpropyl)[1,4'-bipiperidine]-4'-carboxamide (piritramide), profadol, proheptazine, promedol, properidine, propoxyphene, N-(1-methyl-2-piperidinoethyl)-N-(2-pyridyl)-propionamide, methyl {3-[4-methoxycarbonyl-4-(N-phenylpropanamido)piperidino]-propanoate} (remifentanil), N-{4-methoxymethyl-1-[2-(2-thienyl)ethyl]-4-piperidyl}-propionanilide (sufentanil), ethyl (2-dimethylamino-1-phenyl-3-cyclohexene-1-carboxylate) (tilidine (cis and trans)), tramadol, (1R*,2R*)-3-(3-dimethylamino-1-ethyl-2-methyl-propyl)-phenol, (1R,2R,4S)-2-(dimethylamino)methyl-4-(p-fluorobenzyloxy)-1-(m-methoxyphenyl)cyclohexanol, possibly in the form of respective corresponding stereoisomeric compounds and also corresponding derivatives, in particular esters or ethers, and respective physiologically acceptable compounds, in particular salts and solvates.

8. Dosage form according to one of claims 4 to 7, **characterised in that** it contains one or more stimulants selected from the group consisting of amphetamine, norpseudoephedrine, methylphenidate and possibly respective corresponding physiologically acceptable compounds thereof, in particular bases, salts and solvates thereof.

9. Dosage form according to one of claims 1 to 8, **characterised in that** the irritant of component (a) causes burning, itching, an urge to sneeze, increased formation of secretions or a combination of at least two of these irritations.

10. Dosage form according to one of claims 1 to 9, **characterised in that** the irritant of component (a) is based on one or more ingredients of at least one hot substance drug.

11. Dosage form according to claim 10, **characterised in that** the hot substance drug is selected from the group consisting of Allii sativi bulbus, Asari rhizoma cum herba, Calami rhizoma, Capsici fructus (capsicum), Capsici fructus acer (cayenne pepper), Curcumae longae rhizoma, Curcumae xanthorrhizae rhizoma, Galangae rhizoma, Myristicae semen, Piperis nigri fructus (pepper), Sinapis albae semen (white mustard seed), Sinapis nigri semen, Zedoariae rhizoma and Zingiberis rhizoma, particularly preferred from the group consisting of Capsici fructus (capsicum), Capsici fructus acer (cayenne pepper) and Piperis nigri fructus (pepper).

12. Dosage form according to claim 10 or 11, **characterised in that** the ingredient is an o-methoxy(methyl)phenol compound, an acid amide compound, a mustard oil or a sulphide compound or is derived from such a compound.

13. Dosage form according to one of claims 10 to 12, **characterised in that** the ingredient is selected from the group consisting of myristicin, elemicin, isoeugenol, β-asarone, safrole, gingerols, xanthorrhizol, capsaicinoids, preferably capsaicin, piperine, preferably trans-piperine, glucosinolates, preferably based on non-volatile mustard oils, particularly preferred based on p-hydroxybenzyl mustard oil, methylmercapto mustard oil or methylsulphonyl mustard oil, and compounds derived from these ingredients.

14. Dosage form according to one of claims 1 to 13, **characterised in that** component (b) has one or more viscosity-increasing agents selected from the group consisting of microcrystalline cellulose with 11% by wt. carboxymethylcellulose sodium (Avicel^{®} RC 591), carboxymethylcellulose sodium (Blanose^{®}, CMC-Na C300P^{®}, Frimulsion BLC-5^{®}, Tylose C300 P^{®}), polyacrylic acid (Carbopol^{®} 980 NF, Carbopol^{®} 981), locust bean flour (Cesagum^{®} LA-200, Cesagum^{®} LID/150, Cesagum^{®} LN-1), citrus pectin (Cesapectin^{®} HM Medium Rapid Set), waxy maize starch (C*Gel 04201^{®}), sodium alginate (Frimulsion ALG (E401)^{®}) guar flour (Frimulsion BM^{®}, Polygum 26/1-75^{®}), iota-carrageenan (Frimulsion D021^{®}), karaya gum, gellan gum (Kelcogel F^{®}, Kelcogel LT100^{®}), galactomannan (Meyprogat 150^{®}), tara stone flour (Polygum 43/1^{®}), propylene glycol alginate (Protanal-Ester SD-LB^{®}), apple pectin, pectin from citrus peel, sodium hyaluronate, tragacanth, tara gum (Vidogum SP 200^{®}), fermented polysaccharide welan gum (K1A96) and xanthan gum (Xantural 180^{®}).

15. Dosage form according to one of claims 1 to 14, **characterised in that** it contains the viscosity-increasing agents in a quantity of ≥5 mg per dosage form, i.e. per dosage unit.

16. Dosage form according to one of claims 7 to 15, **characterised in that** the antagonist of component (c) used is an opiate or opioid antagonist selected from the group consisting of naloxone, naltrexone, nalmefene, nalide, nalmexone, nalorphine, naluphine and respective corresponding physiologically acceptable compounds, in particular bases, salts and solvates.

17. Dosage form according to one of claims 4 to 16, **characterised in that** the antagonist of component (c) used as a stimulant is a neuroleptic preferably selected from the group consisting of haloperidol, promethazine, fluphenazine, perphenazine, levomepromazine, thioridazine, perazine, chlorpromazine, chlorprothixine, zuclopentixol, flupentixol, prothipendyl, zotepine, benperidol, pipamperone, melperone and bromperidol.

18. Dosage form according to one of claims 1 to 17, **characterised in that** the emetic of component (d) is based on one or more constituents of ipecacuanha (ipecac) root, preferably based on the constituent emetine, and/or apomorphine.

19. Dosage form according to one of claims 1 to 18, **characterised in that** the active ingredient or ingredients is/are spatially separated from components (c) and/or (d), wherein the active ingredient or ingredients is/are preferably present in at least one sub-unit (A) and components (c) and/or (d) are present in one sub-unit (B), and when the dosage form is correctly administered, components (c) and/or (d) from subunit (B) do not become active in the body.

20. Dosage form according to claim 19, **characterised in that** both sub-units (A) and (B) are present in multiparticulate form, preferably in the form of microtablets, microcapsules, micropellets, granules, spheroids, beads or pellets, possibly press-moulded into tablets, packaged in capsules or suspended in a liquid or a gel, wherein the same form, i.e. structure, is selected for both sub-unit (A) and sub-unit (B).

21. Dosage form according to claim 20, **characterised in that** the respective substantially identically structured multiparticulate forms of sub-unit (A) or (B) are likewise not visually distinguishable.

22. Dosage form according to claim 19, **characterised in that** sub-units (A) and (B) are respectively arranged in layered form in relation to one another.

23. Dosage form according to claim 22, **characterised in that** the layered sub-units (A) and (B) are arranged vertically or horizontally to one another.

24. Dosage form according to claim 22, **characterised in that** sub-unit (B) forms a core, which is completely enveloped by sub-unit (A).

25. Dosage form according to claim 22, **characterised in that** sub-unit (A) forms core, which is enveloped by sub-unit (B), wherein this envelope has at least one channel, which leads from the core to the surface of the dosage form.

26. Dosage form according to one of claims 22 to 25, **characterised in that** at least one possibly swellable separation layer (C) is arranged between the layers of sub-units (A) and (B).

27. Dosage form according to claim 22 or 26, **characterised in that** it is present in the form of a tablet.

28. Dosage form according to one of claim 22, 23, 26 or 27, **characterised in that** the free surface of sub-unit (B) is covered completely, and possibly at least one portion of the free surface of sub-unit (A) and possibly at least one portion of the free surface of the separation layer (C) is/are covered with at least one barrier layer (D) that inhibits the release of components (c) and/or (d).

29. Dosage form according to one of claim 22, 23, 26 or 27, **characterised in that** it has a push layer (P) between sub-units (A) and (B) and all the free surfaces of the layered structure composed of sub-units (A) and (B), the push layer (P) and possibly the separation layer (C) are provided with a semipermeable coating (E), which is permeable for the release medium, is substantially impermeable for the active ingredient and for components (c) and/or (d), and wherein in the region of sub-unit (A) this coating (E) has at least one opening for the release of the active ingredient.

30. Dosage form according to claim 19, **characterised in that** sub-unit (A) is in the form of a tablet, the edge face of which and possibly one of its two main faces is covered with at least one barrier layer (D) containing the emetic.

31. Dosage form according to one of claims 1 to 30, **characterised in that** it has at least one active ingredient at least partially in delayed release form.

32. Dosgae form according to one of claims 1 to 31 for oral administration.

33. Dosage form according to claim 32, **characterised in that** it has at least one coating resistant to gastric juices.

34. Dosage form according to one of claims 19 to 33, **characterised in that** it contains components (a) and/or (b) in at least one sub-unit A and/or at least one sub-unit B.

## Revendications

1. Forme d'administration assurée contre un usage abusif, **caractérisée en ce qu'**elle présente, outre une ou plusieurs substances actives présentant un potentiel d'usage abusif, deux des composants suivants a)-d) ou plus :
(a) au moins une substance irritant le nez et/ou la gorge,
(b) au moins un agent qui augmente la viscosité, qui forme dans un extrait obtenu à l'aide d'une quantité minimale nécessaire d'un liquide aqueux à partir de la forme d'administration un gel, qui reste visuellement détectable lors de l'introduction dans une autre quantité de liquide aqueux,
(c) au moins un antagoniste de la substance active ou des substances actives présentant un potentiel d'usage abusif
(d) au moins un émétique.

2. Forme d'administration selon la revendication 1, **caractérisée en ce qu'**elle présente trois des composants (a)-(d), de préférence (a), (b) et (c) ou (a), (b) et (d).

3. Forme d'administration selon la revendication 1, **caractérisée en ce qu'**elle présente tous les composants (a)-(d).

4. Forme d'administration selon la revendication 1 ou 2, contenant deux ou trois des composants (a), (b) et (d), **caractérisée en ce que** la substance active est choisie dans le groupe constitué par les opiacés, les opioïdes, les tranquillisants, les stimulants et d'autres anesthésiants.

5. Forme d'administration selon la revendication 4, **caractérisée en ce que** la substance active est choisie dans le groupe constitué par le N-{1-[2-(4-éthyl-5-oxo-2-tétrazolin-1-yl)éthyl]-4-méthoxyméthyl-4-pipéridyl}propionanilide (alfentanil), l'acide 5,5-diallylbarbiturique (allobarbital), l'allylprodine, l'alphaprodine, la 8-chloro-1-méthyl-6-phényl-4H-[1,2,4]triazolo[4,3-a][1,4]-benzodiazépine (alprazolam), la 2-diéthylaminopropiophénone (amfépramone), la . (±)-α-méthylphénéthylamine (amphétamine), le 2-(α-méthylphénéthylamino)-2-phénylacétonitrile (amphétaminil), l'acide 5-éthyl-5-isopentylbarbiturique (amobarbital), l'aniléridine, l'apocodéine, l'acide 5,5-diéthylbarbiturique (barbital), la benzylmorphine, le bézitramide, la 7-bromo-5-(2-pyridyl)-1H-1,4-benzodiazépin-2(3H)-one (bromazépam), la 2-bromo-4-(2-chlorophényl)-9-méthyl-6H-thiéno[3,2-f][1,2,4]triazolo[4,3-a][1,4]diazépine (brotizolam), le 17-cyclopropylméthyl-4,5α-époxy-7α[(S)-1-hydroxy-1,2,2-triméthylpropyl]-6-méthoxy-6,14-endo-éthanomorphinan-3-ol (buprénorphine), l'acide 5-butyl-5-éthylbarbiturique (butobarbital), le butorphanol, le (7-chloro-1,3-dihydro-1-méthyl-2-oxo-5-phényl-2H-1,4-benzodiazépin-3-yl)-diméthylcarbamate (camazépam), le (1S,2S)-2-amino-1-phényl-1-propanol (cathine/D-norpseudoéphédrine), le 7-chloro-N-méthyl-5-phényl-3H-1,4-benzodiazépin-2-ylamin-4-oxyde (chlordiazépoxyde), la 7-chloro-1-méthyl-5-phényl-1H-1,5-benzodiazépine-2,4(3H,5H)-dione (clobazam), la 5-(2-chlorophényl)-7-nitro-1H-1,4-benzodiazépin-2(3H)-one (clonazépam), le clonitazène, l'acide 7-chloro-2,3-dihydro-2-oxo-5-phényl-1 H-1,4-benzodiazépine-3-carboxylique (clorazépate), la 5-(2-chlorophényl)-7-éthyl-1-méthyl-1H-thiéno[2,3-e][1,4]diazépin-2(3H)-one (clotiazépam), la 10-chloro-11b-(2-chlorophényl)-2,3,7,11 b-tétrahydrooxazolo[3,2-d][1,4]benzodiazépin-6(5H)-one (cloxazolam), le (-)-[3β-benzoyloxy-2β(1αH,5αH)-tropanecarboxylate] de méthyle (cocaïne), le 4,5α-époxy-3-méthoxy-17-méthyl-7-morphinen-6α-ol (codéine), l'acide 5-(1-cyclohexényl)-5-éthylbarbiturique (cyclobarbital), le cyclorphan, la cyprénorphine, la 7-chloro-5-(2-chlorophényl)-1H-1,4-benzodiazépin-2(3H)-one (délorazépam), la désomorphine, le dextromoramide, le propionate de (+)-(1-benzyl-3-diméthylamino-2-méthyl-1-phénylpropyle) (dextropropoxyphène), la dézocine, le diampromide, la diamorphone, la 7-chloro-1-méthyl-5-phényl-1H-1,4-benzodiazépine-2(3H)-one (diazépam), le 4,5α-époxy-3-méthoxy-17-méthyl-6α-morphinanol (dihydrocodéine), le 4,5□-époxy-17-méthyl-3,6a-morphinanediol (dihydromorphine), le dimenoxadol, le diméphétamol, le diméthylthiambutène, le dioxaphetylbutyrate, la dipipanone, le (6aR,10aR)-6,6,9-triméthyl-3-pentyl-6a,7,8,10a-tétrahydro-6H-benzo[c]chromén-1-ol (dronabinol), l'eptazocine, la 8-chloro-6-phényl-4H-[1,2,4]triazolo[4,3-a][1,4]benzodiazépine (estazolam), l'éthoheptazine, l'éthylméthylthiambutène, le [7-chloro-5-(2-fluorophényl)-2,3-dihydro-2-oxo-1H-1,4- benzodiazépine-3-carboxylate] d'éthyle (l'éthylloflazépate), le 4,5□-époxy-3-éthoxy-17 -méthyl-7 -morphinén-6□-ol (éthylmorphine), l'étonitazène, le 4,5α-époxy-7α-(1-hydroxy-1-méthylbutyl)-6-méthoxy-17-méthyl-6,14-endo-éthénomorphinan-3-ol (étorphine), la N-éthyl-3-phényl-8,9,10-trinorbornan-2-ylamine (fencamfamine), la 7-[2-(α-méthylphénéthylamino)éthyl]-théophylline) (fénétylline), le 3-(α-méthylphénéthylamino)propionitrile (fenproporex), le N-(1-phénéthyl-4-pipéridyl)propionanilide (fentanyl), la 7-chloro-5-(2-fluorophényl)-1-méthyl-1H-1,4-benzodiazépin-2(3H)-one (fludiazépam), la 5-(2-fluorophényl)-1-méthyl-7-nitro-1H-1,4-benzodiazépin-2(3H)-one (flunitrazépam), la 7-chloro-1-(2-diéthylaminoéthyl)-5-(2-fluorophényl)-1H-1,4-benzodiazépin-2(3H)-one (flurazépam), la 7-chloro-5-phényl-1-(2,2,2-trifluoréthyl)-1H-1,4-benzodiazépin-2(3H)-one (halazépam), la 10-bromo-11b-(2-fluorophényl)-2,3,7,11 b-tétrahydro[1,3]oxazolo[3,2-d][1,4]benzodiazépine-6(5H)-one (haloxazolam), l'héroïne, la 4,5α-époxy-3-méthoxy-17-méthyl-6-morphinanone (hydrocodone), la 4,5α-époxy-3-hydroxy-17-méthyl-6-morphinanone (hydromorphone), l'hydroxypéthidine, l'isométhadone, l'hydroxyméthylmorphinane, la 11-chloro-8,12b-dihydro-2,8-diméthyl-12b-phényl-4H-[1,3]oxazino[3,2-d][1,4]benzodiazépin-4,7(6H)-dione (kétazolam), la 1-[4-(3-hydroxyphényl)-1-méthyl-4-pipéridyl]-1-propanone (ketobemidon), l'acétate de (3S,6S)-6-diméthylamino-4,4-diphénylheptan-3-yle (lévacétylméthadol (LAAM)), la (-)-6-diméthylamino-4,4-diphényl-3-heptanone (lévométhadone), le (-)-17-méthyl-3-morphinanol (lévorphanol), le lévophénacylmorphane, le lofentanil, la 6-(2-chlorophényl)-2-(4-méthyl-1-pipérazinylméthylène)-8-nitro-2H-imidazo[1,2-a][1,4]benzodiazépin-1(4H)-one (loprazolam), la 7-chloro-5-(2-chlorophényl)-3-hydroxy-1H-1,4-benzodiazépin-2(3H)-one (lorazépam), la 7-chloro-5-(2-chlorophényl)-3-hydroxy-1-méthyl-1H-1,4-benzodiazépin-2(3H)-one(lormétazépam), le 5-(4-chlorophényl)-2,5-dihydro-3H-imidazo[2,1-a]iso-indol-5-ol(mazindol), la 7-chloro-2,3-dihydro-1-méthyl-5-phényl-1H-1,4-benzodiazépine (médazépam), la N-(3-chloropropyl)-α-méthylphénéthylamine (méfénorex), la mépéridine, le 2-méthyl-2-propyltriméthylènedicarbamate (le méprobamate), le meptazinol, la métazocine, la méthylmorphine, la N,α-diméthylphénéthylamine (metamphétamine), la (±)-6-diméthylamino-4,4-diphényl-3-heptanone (méthadone), la 2-méthyl-3-o-toluyl-4(3H)-quinazolinone (méthaqualone), le [2-phényl-2-(2-pipéridyl)acétate] de méthyle (méthylphénidate), l'acide 5-éthyl-1-méthyl-5-phénylbarbiturique (méthylphénobarbital), la 3,3-diéthyl-5-méthyl-2,4-pipéridinedione (le méthyprylon), la métopone, la 8-chloro-6-(2-fluorophényl)-1-méthyl-4H-imidazo[1,5-a][1,4]benzodiazépine (midazolam), le 2-(benzhydrylsulfinyl)acétamide (modafinil), le 4,5α-époxy-17-méthyl-7-morphinène-3,6α-diol (morphine), la myrophine, la (±)-trans-3-(1,1-diméthylheptyl)-7,8,10,10α-tétrahydro-1-hydroxy-6,6-diméthyl-6H-dibenzo[b,d]pyran-9(6αH)-one (nabilon), la nalbuphène, la nalorphine, la narcéine, la nicomorphine, la 1-méthyl-7-nitro-5-phényl-1H-1,4-benzodiazépin-2(3H)-one (nimétazépam), la 7-nitro-5-phényl-1H-1,4-benzodiazépin-2(3H)-one (nitrazépam), la 7-chloro-5-phényl-1H-1,4-benzodiazépin-2(3H)-one (nordazépam), le norlévorphanol, la 6-diméthylamino-4,4-diphényl-3-hexanone (norméthadone), la normorphine, la norpipanone, le jus coagulé des plantes appartenant à l'espèce Papaver somniferum (opium), la 7-chloro-3-hydroxy-5-phényl-1H-1,4-benzodiazépin-2(3H)-one (oxazépam), la (cis-trans)-10-chloro-2,3,7,11b-tétrahydro-2-méthyl-11b-phényloxazolo[3,2-d][1,4]benzodiazépin-6-(5H)-one (oxazolam), la 4,5α-époxy-14-hydroxy-3-méthoxy-17-méthyl-6-morphinanone (oxycodone), l'oxymorphone, les plantes et parties de plantes appartenant à l'espèce Papaver somniferum (y compris la sous-espèce setigerum) (Papaver somniferum), le papaveretum, 2-imino-5-phényl-4-oxazolidinone (pernoline), le 1,2,3,4,5,6-hexahydro-6,11-diméthyl-3-(3-méthyl-2-butényl)-2,6-méthano-3-benzazocin-8-ol (pentazocine), l'acide 5-éthyl-5-(1-méthylbutyl)-barbiturique (pentobarbital), le (1-méthyl-4-phényl-4-pipéridinecarboxylate) d'éthyle (péthidine), la phénadoxone, le phénomorphane, la phénazocine, la phénopéridine, la piminodine, la pholcodéine, la 3-méthyl-2-phénylmorpholine (phénmetrazine), l'acide 5-éthyl-5-phénylbarbiturique (phénobarbital), l'α,α-diméthylphénéthylamine (phéntermine), la 7-chloro-5-phényl-1-(2-propynyl)-1H-1,4-benzodiazépin-2(3H)-one (pinazépam), l'alcool α-(2-pipéridyl)benzhydrylique (pipradrol), le 1'-(3-cyano-3,3-diphénylpropyl)[1,4'-bipipéridine]-4'-carboxamide (piritramide), la 7-chloro-1-(cyclopropylméthyl)-5-phényl-1H-1,4-benzodiazépin-2(3H)-one (prazépam), le profadol, la proheptazine, le promédol, la propéridine, le propoxyphène, le N-(1-méthyl-2-pipéridinoéthyl)-N-(2-pyridyl)propionamide, le {3-[4-méthoxycarbonyl-4-(N-phénylpropanamido)pipéridino]propanoate} de méthyle (rémifentanil), l'acide 5-sec-butyl-5-éthylbarbiturique (secbutabarbital), l'acide 5-allyl-5-(1-méthylbutyl)-barbiturique (secobarbital), le N-{4-méthoxyméthyl-1-[2-(2-thiényl)éthyl]-4-pipéridyl)propionanilide (sufentanil), la 7-chloro-2-hydroxyméthyl-5-phényl-1H-1,4-benzodiazépin-2(3H)-one (témazépam), la 7-chloro-5-(1-cyclohexényl)-1-méthyl-1H-1,4-benzodiazépin-2(3H)-one (tétrazépam), le (2-diméthylamino-1-phényl-3-cyclohexène-1-carboxylate) d'éthyle (tilidine (cis et trans)), le tramadol, la 8-chloro-6-(2-chlorophényl)-1-méthyl-4H-[1,2,4]triazolo[4,3-a][1,4]benzodiazépine (triazolam), l'acide 5-(1-méthylbutyl)-5-vinylbarbiturique (vinylbital), le (1R*,2R*)-3-(3-diméthylamino-1-éthyl-2-méthylpropyl)-phénol, le (1R,2R,4S)-2-[diméthylamino)méthyl-4-(p-fluorobenzyloxy)-1-(m-méthoxyphényl)cyclohexanol, à chaque fois le cas échéant sous forme des composés stéréo-isomères correspondants ainsi que des dérivés correspondants, en particulier des esters ou des éthers, et à chaque fois leurs composés physiologiquement acceptables, en particulier les sels et les solvates.

6. Forme d'administration selon l'une quelconque des revendications 1 à 3 contenant le composant (c), **caractérisée en ce que** la substance active est choisie dans le groupe constitué par les opiacés, les opioïdes, les stimulants et d'autres agents anesthésiants.

7. Forme d'administration selon la revendication 6, **caractérisée en ce que** la substance active a été choisie dans le groupe constitué par le N-{1-[2-(4-éthyl-5-oxo-2-tétrazolin-1-yl)éthyl]-4-méthoxyméthyl-4-pipéridyl}propionanilide (alfentanil), l'allylprodine, l'alphaprodine, la 2-diéthylaminopropiophénone (amfépramone), la (±)-α-méthylphénéthylamine (amphétamine), le 2-(α-méthylphénéthylamino)-2-phénylacétonitrile (amphétaminil), l'aniléridine, l'apocodéine, la benzylmorphine, le bézitramide, le 17-cyclopropylméthyl-4,5α-époxy-7α[(S)-1-hydroxy-1,2,2-triméthylpropyl]-6-méthoxy-6,14-endo-éthanomorphinan-3-ol (buprénorphine), le butorphanol, le (1S,2S)-2-amino-1-phényl-1-propanol (cathine/D-norpseudoéphédrine), le clonitazène, le (-)-[3β-benzoyloxy-2β(1αH,5αH)-tropanecarboxylate] de méthyle (cocaïne), le 4,5α-époxy-3-méthoxy-17-méthyl-7-morphinén-6α-ol (codéine), le cyclorphane, la cyprénorphine, la désomorphine, le dextromoramide, le propionate de (+)-(1-benzyl-3-diméthylamino-2-méthyl-1-phénylpropyle)(dextropropoxyphène), la dézocine, le diampromide, la diamorphone, le 4,5α-époxy-3-méthoxy-17-méthyl-6α-morphinanol (dihydrocodéine), le 4,5□-époxy-17-méthyl-3,6α-morphinanediol (dihydromorphine), le diménoxadol, le diméphétamol, le diméthylthiambutène, le butyrate de dioxaphétyle, la dipipanone, le (6aR, 10aR)-6,6,9-triméthyl-3-pentyl-6a,7,8,10a-tétrahydro-6H-benzo[c]chromén-1-ol (dronabinol), l'eptazocine, l'éthoheptazine, l'éthylméthylthiambutène, le 4,5□-époxy-3-éthoxy-17-méthyl-7-morphinén-6□-ol (éthylmorphine), l'étonitazène, le 4,5α-époxy-7α-(1-hydroxy-1-méthylbutyl)-6-méthoxy-17-méthyl-6,14-endo-éthénomorphinan-3-ol (étorphine), la N-éthyl-3-phényl-8,9,10-trinorbornan-2-ylamine (fencamfamine), la 7-[2-(α-méthylphénéthylamino)éthyl]-théophylline) (fénétylline), le 3-(α-méthylphénéthylamino)propionitrile (fenproporex), le N-(1-phénéthyl-4-pipéridyl)propionanilide (fentanyl), l'héroïne, la 4,5α-époxy-3-méthoxy-17-méthyl-6-morphinanone (l'hydrocodone), la 4,5α-époxy-3-hydroxy-17-méthyl-6-morphinanone (hydromorphone), l'hydroxypéthidine, l'isométhadone, le hydroxyméthylmorphinane, la 1-[4-(3-hydroxyphényl)-1-méthyl-4-pipéridyl]-1-propanone (ketobemidone), l'acétate de (3S,68)-6-diméthylamino-4,4-diphénylheptan-3-yle (lévacétylméthadol (LAAM)), la (-)-6-diméthylamino-4,4-diphényl-3-heptanone (lévométhadone), le (-)-17-méthyl-3-morphinanol (lévorphanol), le lévophénacylmorphane, le lofentanil, le 5-(4-chlorophényl)-2,5-dihydro-3H-imidazo[2,1-a]iso-indol-5-ol (mazindol), la N-(3-chloropropyl)-α-méthylphénéthylamine (méfénorex), la mépéridine, le meptazinol, la métazocine, la méthylmorphine, la N,α-diméthylphénéthylamine (métamphétamine), la (±)-6-diméthylamino-4,4-diphényl-3-heptanone (méthadone), le [2-phényl-2-(2-pipéridyl)acétate] de méthyle (méthylphénidate), la 3,3-diéthyl-5-méthyl-2,4-pipéridinedione (méthyprylon), le 2-(benzhydrylsulfinyl)acétamide (modafinil), le 4,5α-époxy-17-méthyl-7-morphinène-3,6α-diol (morphine), la myrophine, la (±)-trans-3-(1,1-diméthylheptyl)-7,8,10,10α-tétrahydro-1-hydroxy-6,6-diméthyl-6H-dibenzo[b,d]pyran-9(6αH)-one (nabilon), le nalbuphène, la narcéine, la nicomorphine, le norlévorphanol, la 6-diméthylamino-4,4-diphényl-3-hexanone (norméthadone), la normorphine, la norpipanone, le jus coagulé des plantes appartenant à l'espèce Papaver somniferum (opium), la 4,5α-époxy-14-hydroxy-3-méthoxy-17-méthyl-6-morphinanone (oxycodon), l'oxymorphone, les plantes et parties des plantes appartenant à l'espèce Papaver somniferum (y compris la sous-espèce setigerum) (Papaver somniferum), le papaveretum, la 2-imino-5-phényl-4-oxazolidinone (pernoline), le 1,2,3,4,5,6-hexahydro-6,11-diméthyl-3-(3-méthyl-2-butényl)-2,6-méthano-3-benzazocin-8-ol (pentazocine), le (1-méthyl-4-phényl-4-pipéridinecarboxylate) d'éthyle (péthidine), la phénadoxone, le phénomorphane, la phénazocine, la phénopéridine, la piminodine, la pholcodéine, la 3-méthyl-2-phénylmorpholine (phénmétrazine), l'α,α-diméthylphénéthylamine (phéntermine), l'alcool α-(2-pipéridyl)benzhydrylique (pipradrol), le 1'-(3-cyano-3,3-diphénylpropyl)[1,4'-bipipéridine]-4'-carboxamide (piritramide), le profadol, la proheptazine, le promédol, la propéridine, le propoxyphène, le N-(1-méthyl-2-pipéridinoéthyl)-N-(2-pyridyl)propionamide, le {3-[4-méthoxycarbonyl-4-(N-phénylpropanamido)pipéridino]propanoate} de méthyle (rémifentanil), le N-{4-méthoxyméthyl-1-[2-(2-thiényl)éthyl]-4-pipéridyl)propionanilide (sufentanil), le (2-diméthylamino-1-phényl-3-cyclohexène-1-carboxylate) d'éthyle (tilidine (cis et trans)), le tramadol, le (1R*,2R*)-3-(3-diméthylamino-1-éthyl-2-méthylpropyl)-phénol, le (1R,2R,4S)-2-[diméthylamino)méthyl-4-(p-fluorobenzyloxy)-1-(m-méthoxyphényl)cyclohexanol, à chaque fois le cas échéant sous forme des composés stéréoisomères correspondants ainsi que des dérivés correspondants, en particulier des esters ou des éthers et à chaque fois leurs composés physiologiquement acceptables, en particulier les sels et les solvates.

8. Forme d'administration selon l'une quelconque des revendications 4 à 7, **caractérisée en ce qu'**elle contient un ou plusieurs stimulants, choisis dans le groupe formé par l'amphétamine, la norpseudoéphédrine, le méthylphénidate et à chaque fois le cas échéant leurs composés physiologiquement acceptables correspondants, en particulier leurs bases, sels et solvates.

9. Forme d'administration selon l'une quelconque des revendications 1 à 8, **caractérisée en ce que** la substance irritante du composant (a) provoque une brûlure, une démangeaison, une irritation sternutoire, une sécrétion accrue ou une combinaison d'au moins deux de ces irritations.

10. Forme d'administration selon l'une quelconque des revendications 1 à 9, **caractérisée en ce que** la substance irritante du composant (a) est à base d'un ou de plusieurs constituants d'au moins un extrait piquant.

11. Forme d'administration selon la revendication 10, **caractérisée en ce que** l'extrait piquant est choisi dans le groupe constitué par Allii sativi Bulbus, Asari Rhizoma c. Herbe, Calami Rhizoma, Capsici Fructus (Paprika), Capsici Frucfus acer (poivre de Cayenne), Curcumae longae Rhizoma, Curcumae xanthorrhizae Rhizoma, Galangae Rhizoma, Myristicae Semen, Piperis nigri Fructus (poivre), Sinapis albae (Erucae) Semen, Sinapis nigri Semen, Zedoariae Rhizoma et Zngiberis Rhizoma, de manière particulièrement préférée dans le groupe constitué par Capsici Fructus (Paprika), Capsici Fructus acer (poivre de Cayenne) et Piperis nigri Fructus (poivre).

12. Forme d'administration selon la revendication 10 ou 11, **caractérisée en ce que** le constituant est un composé du type o-méthoxy(méthyl)-phénol, un composé du type amide d'acide, une huile de moutarde ou un composé de type sulfure ou est dérivé d'un tel composé.

13. Forme d'administration selon l'une quelconque des revendications 10 à 12, **caractérisée en ce que** le constituant a été choisi dans le groupe comprenant la myristicine, l'élémicine, l'isoeugénol, la β-asarone, le safrol, les gingérols, le xanthorrhizol, les capsaïcinoïdes, de préférence la capsaïcine, la pipérine, de préférence la trans-pipérine, les glucosinolates, de préférence à base d'huiles de moutarde non volatiles, de manière particulièrement préférée à base de p-hydroxybenzyl-huile de moutarde, de méthylmercapto-huile de moutarde ou de méthylsulfonyl-huile de moutarde, et les composés dérivés de ces constituants.

14. Forme d'administration selon l'une quelconque des revendications 1 à 13, **caractérisée en ce que** le composé (b) présente un ou plusieurs agents augmentant la viscosité, choisis dans le groupe constitué par la cellulose microcristalline comprenant 11% en poids de carboxyméthylcellulose sodique (Avicel® RC 591), la carboxyméthylcellulose sodique (Blanose®, CMC-Na C300P®, Frimulsion BLC-5®, Tylose C300 P®), le poly(acide acrylique) (Carbopol® 980 NF, Carbopol® 981), la farine de noyaux de caroube (Cesagum® LA-200, Cesagum® LID/150, Cesagum® LN-1), la pectine d'agrumes (Cesapectin® HM Medium Rapid Set), l'amidon de maïs cireux (C*Gel 04201®), l'alginate de sodium (Frimulsion ALG (E401)®), la farine de graines de Guar (Frimulsion BM®, Polygum 26/1-75®), le carraghénane Iota (Frimulsion D021®), la gomme Karaya, la gomme gellane (Kelcogel F®, Kelcogel LT100®), le galactomannane (Meyprogat 150®), la farine de graines de tara (Polygum 43/1®), l'alginate de propylèneglycol (Protanal-Ester SD-LB®), la pectine de pomme, la pectine d'écorces de citron, l'hyaluronate de sodium, la gomme adragante, la gomme de tara (Vidogum SP 200®), la gomme de welan-polysaccharide fermenté (K1A96) et la gomme de xanthane (Xantural 180®).

15. Forme d'administration selon l'une quelconque des revendications 1 à 14, **caractérisée en ce qu'**elle présente les agents augmentant la viscosité en une quantité de ≥ 5 mg par forme d'administration, c'est-à-dire par unité de dosage.

16. Forme d'administration selon l'une quelconque des revendications 7 à 15, **caractérisée en ce qu'**on utilise, comme antagoniste du composant (c), un antagoniste d'opiacé ou d'opioïde choisi dans le groupe comprenant la naloxone, la naltrexone, le nalmefene, le nalide, le nalmexone, la nalorphine, la naluphine et à chaque fois des composés correspondants, physiologiquement acceptables, en particulier les bases, les sels ou les solvates.

17. Forme d'administration selon l'une quelconque des revendications 4 à 16, **caractérisée en ce qu'**on utilise, comme antagoniste du composant (c) pour un stimulant, un neuroleptique, de préférence choisi dans le groupe formé par l'halopéridol, la prométhacine, la fluphénazine, la perphénazine, la lévomépromazine, la thioridazine, la pérazine, la chlorpromazine, la chlorprothixine, le zuclopenthixol, le flupentixol, la prothipendyle, la zotépine, le benpéridol, la piparmpérone, le melpérone et le brompéridol.

18. Forme d'administration selon l'une quelconque des revendications 1 à 17, **caractérisée en ce que** l'émétique du composant (d) est à base d'un ou de plusieurs constituants du Radix Ipecacuanhae (racine d'ipéca), de préférence du constituant émétine et/ou est l'apomorphine.

19. Forme d'administration selon l'une quelconque des revendications 1 à 18, **caractérisée en ce que** la substance active ou les substances actives des composants (c) et/ou (d) est/sont séparée(s) dans l'espace, la substance active ou les substances actives se trouvant de préférence dans au moins une sous-unité (A) et les composants (c) et/ou (d) se trouvant dans au moins une sous-unité (B) et les composants (c) et/ou (d) de la sous-unité (B) ne déployant pas leur effet dans le corps lors d'une administration selon les dispositions en vigueur.

20. Forme d'administration selon la revendication 19, **caractérisée en ce que** les deux sous-unités (A) et (B) se trouvent sous forme microparticulaire, de préférence sous forme de microcomprimés, de microcapsules, de micropellets, de granulats, de sphères, de billes ou de pellets, le cas échéant, sous forme pressée en comprimés, transvasée dans des capsules ou en suspension dans un liquide ou un gel, en choisissant tant pour la sous-unité (A) que pour la sous-unité (B) la même forme c'est-à-dire le même aspect.

21. Forme d'administration selon la revendication 20, **caractérisée en ce que** les formes multiparticulaires à chaque fois dans une large mesure identiques de la sous-unité (A) ou (B) ne peuvent pas non plus être distinguées visuellement.

22. Forme d'administration selon la revendication 19, **caractérisée en ce que** les sous-unités (A) et (B) sont à chaque fois disposées en couches l'une sur l'autre.

23. Forme d'administration selon la revendication 22, **caractérisée en ce que** les sous-unités (A) et (B) en forme de couches sont assemblées horizontalement ou verticalement l'une par rapport à l'autre.

24. Forme d'administration selon la revendication 22, **caractérisée en ce que** la sous-unité (B) forme un noyau qui est complètement enrobé par la sous-unité (A).

25. Forme d'administration selon la revendication 22, **caractérisée en ce que** la sous-unité (A) forme un noyau qui est enrobé dans la sous-unité (B), cet enrobage présentant au moins un canal qui conduit du noyau à la surface de la forme d'administration, ou

26. Forme d'administration selon l'une quelconque des revendications 22 à 25, **caractérisée en ce qu'**entre les couches des sous-unités (A) et (B) est disposée au moins une couche de séparation (C) le cas échéant gonflable.

27. Forme d'administration selon la revendication 22 ou 26, **caractérisée en ce qu'**elle se trouve sous forme d'un comprimé.

28. Forme d'administration selon l'une quelconque des revendications 22, 23, 26 ou 27, **caractérisée en ce que** la surface libre de la sous-unité (B) est revêtue complètement et le cas échéant au moins une partie de la surface libre de la sous-unité (A) et le cas échéant au moins une partie de la surface libre de la couche de séparation (C) sont revêtues par au moins une couche-barrière (D) empêchant la libération du composant (c) et/ou (d).

29. Forme d'administration selon l'une quelconque des revendications 22, 23, 26 ou 27, **caractérisée en ce qu'**elle présente entre les sous-unités (A) et (B) une couche à effet poussant (P) et toutes les surfaces libres de la structure à couches constituée par les sous-unités (A) et (B), la couche à effet poussant (P) et le cas échéant la couche de séparation (C), présentent un revêtement (E) semi-perméable, qui est perméable à l'agent de libération, mais sensiblement imperméable pour la substance active et pour les composants (c) et/ou (d) et ce revêtement (E) présentant dans la zone de la sous-unité (A) au moins une ouverture pour la libération de la substance active.

30. Forme d'administration selon la revendication 19, **caractérisée en ce que** la sous-unité (A) a la forme d'un comprimé dont l'âme et le cas échéant une des deux surfaces de base est revêtue par au moins une couche-barrière (D) contenant l'émétique.

31. Forme d'administration selon l'une quelconque des revendications 1 à 30, **caractérisée en ce qu'**elle présente au moins une substance active au moins partiellement dans une forme retard.

32. Forme d'administration selon l'une quelconque des revendications 1 à 31 destinée à une administration par voie orale.

33. Forme d'administration selon la revendication 32, **caractérisée en ce qu'**elle présente au moins un revêtement résistant au suc gastrique.

34. Forme d'administration selon l'une quelconque des revendications 19 à 33, **caractérisée en ce qu'**elle contient le composant (a) et/ou le composant (b) dans au moins une sous-unité A et/ou au moins une sous-unité B.
